# EUROPEAN PATENT APPLICATION

(11) **EP 4 269 621 A2**
(43) Date of publication of application: **01.11.2023**
(21) Application number: 23188375.2
(22) Date of filing: 18.07.2018
(51) Int. Cl.: C12Q 1/6883

(54) **MIRNAS AS BIOMARKERS FOR PARKINSON'S DISEASE**

(30) Priority: 20.07.2017 EP 17182387
(62) Divisional of application: 18743758.7
(71) Applicant: Hummingbird Diagnostics GmbH, 69120 Heidelberg (DE)
(72) Inventor: KELLER, Andreas, 66123 Hassel (DE); KAHRAMAN, Mustafa, 66111 Saarbrücken (DE); LAUFER, Thomas, 68535 Edingen-Neckarhausen (DE); KOHLHAAS, Jochen, 69121 Heidelberg (DE)
(74) Representative: Geling, Andrea

(57) **Abstract**

The present invention relates to a method for diagnosing Parkinson's disease (PD) in an individual. Further, the present invention relates to a method for determining the course of Parkinson's disease (PD) in an individual having PD. Furthermore, the present invention relates to a method for determining the risk for developing Parkinson's disease (PD) in an individual. In addition, the present invention relates to the use of at least one polynucleotide for detecting at least one miRNA in a biological sample isolated from an individual for diagnosing Parkinson's disease (PD) in the individual, the use of at least one polynucleotide for detecting at least one miRNA in a biological sample isolated from an individual having Parkinson's disease (PD) for determining the course of PD in the individual, and/or the use of at least one polynucleotide for detecting at least one miRNA in a biological sample isolated from an individual for determining the risk of the individual to develop Parkinson's disease (PD). Moreover, the present invention relates to a kit for diagnosing Parkinson's disease (PD) in an individual, for determining the course of Parkinson's disease (PD) in an individual having PD, and/or for determining the risk for developing Parkinson's disease (PD) in an individual.

## Description

The present invention relates to a method for diagnosing Parkinson's disease (PD) in an individual. Further, the present invention relates to a method for determining the course of Parkinson's disease (PD) in an individual having PD. Furthermore, the present invention relates to a method for determining the risk for developing Parkinson's disease (PD) in an individual. In addition, the present invention relates to the use of at least one polynucleotide for detecting at least one miRNA in a biological sample isolated from an individual for diagnosing Parkinson's disease (PD) in the individual, the use of at least one polynucleotide for detecting at least one miRNA in a biological sample isolated from an individual having Parkinson's disease (PD) for determining the course of PD in the individual, and/or the use of at least one polynucleotide for detecting at least one miRNA in a biological sample isolated from an individual for determining the risk of the individual to develop Parkinson's disease (PD). Moreover, the present invention relates to a kit for diagnosing Parkinson's disease (PD) in an individual, for determining the course of Parkinson's disease (PD) in an individual having PD, and/or for determining the risk for developing Parkinson's disease (PD) in an individual.

### BACKGROUND OF THE INVENTION

Molecular diagnostics has increasingly gained in importance. It has found an entry into the clinical diagnosis of diseases (inter alia detection of infectious pathogens, detection of mutations of the genome, detection of diseased cells and identification of risk factors for predisposition to a disease). In particular, through the determination of gene expression in biological samples such as bodily fluids and tissues, nucleic acid analysis opens up very promising new possibilities in the study and diagnosis of diseases.

Nucleic acids of interest to be detected include genomic DNA, expressed mRNA and other RNAs such as microRNAs (abbreviated miRNAs). MiRNAs are a new class of small RNAs with various biological functions. They are short (average of 20-24 nucleotide) ribonucleic acid (RNA) molecules found in eukaryotic cells. Several hundred different species of miRNAs (i.e. several hundred different sequences) have been identified in mammals. They are important for posttranscriptional gene-regulation and bind to complementary sequences on target messenger RNA transcripts (mRNAs), which can lead to translational repression or target degradation and gene silencing. As such they can also be used as biologic markers for research, diagnosis, and therapy purposes.

Parkinson's disease (PD) is a neurodegenerative disease of the central nervous system which develops with high frequency with aging, and the incidence rate is more than 1% of the population aged 65 and over. It is anticipated that the number of patients with Parkinson's disease will significantly increase in association with the aging of the population in the future.

The motor symptoms of Parkinson's disease result from the death of dopamine-generating cells in the nervous system; the cause of this cell death is unknown. Early symptoms of Parkinson disease are often mistaken to be age-related problems. Parkinson's disease affects movement, producing motor symptoms and may cause mood, cognition, behavior or thought alterations. Diagnosis of Parkinson's disease is based on the medical history and a neurological examination. Imaging modalities are sometimes used to rule out other disorders. Symptoms, such as frailty and motor symptoms, can be similar to other neurological disorders. Diagnosis can be time consuming, expensive and difficult. In particular, the reliable diagnosis of Parkinson based on non-invasive molecular biomarkers remains a challenge.

Therefore, there exists still an unmet clinical need for an efficient, simple, reliable diagnostic test for Parkinson's disease - especially in the early stages of the disease or before the disease becomes clinically manifested. A second clinical need is to guide the therapy and to monitor the disease status of patients.

The present invention meets these needs. The present inventors found that miRNAs and surprisingly also other small non-coding RNA molecules that have been recently discovered by the inventors are significantly dysregulated in biological samples from patients suffering from Parkinson's disease compared to healthy controls. For simplicity, known miRNAs and the newly discovered small non-coding RNAs that are in big parts miRNAs but can contain other RNA species such as piRNAs are jointly denoted as miRNAs. Thus, miRNAs are appropriated non-invasive biomarkers for diagnosis of Parkinson's disease. In particular, the present inventors identified single miRNAs which allow to determine diagnosis Parkinson's disease with high diagnostic power.

### SUMMARY OF THE INVENTION

In a first aspect, the present invention relates to a method for diagnosing Parkinson's disease (PD) in an individual (suspected of having PD) comprising the step of:
determining the level of at least one miRNA selected from the group consisting of SEQ ID NO: 1 to SEQ ID NO: 702 and a sequence having at least 90% sequence identity thereto in a biological sample isolated from the individual (suspected of having PD).

In a second aspect, the present invention relates to a method for determining the course of Parkinson's disease (PD) in an individual having PD comprising the step of:
determining the level of at least one miRNA selected from the group consisting of SEQ ID NO: 1 to SEQ ID NO: 702 and a sequence having at least 90% sequence identity thereto in a biological sample isolated from the individual (having PD).

In a third aspect, the present invention relates to a method for determining the risk for developing Parkinson's disease (PD) in an individual comprising the step of:
determining the level of at least one miRNA selected from the group consisting of SEQ ID NO: 1 to SEQ ID NO: 702 and a sequence having at least 90% sequence identity thereto in a biological sample isolated from the individual.

In a fourth aspect, the present invention relates to the use of at least one polynucleotide for detecting at least one miRNA in a biological sample isolated from an individual (suspected of having PD) for diagnosing Parkinson's disease (PD) in the individual (suspected of having PD), wherein the at least one miRNA is selected from the group consisting of SEQ ID NO: 1 to SEQ ID NO: 702.

In a fifth aspect, the present invention relates to the use of at least one polynucleotide for detecting at least one miRNA in a biological sample isolated from an individual having Parkinson's disease (PD) for determining the course of PD in the individual (having PD), wherein the at least one miRNA is selected from the group consisting of SEQ ID NO: 1 to SEQ ID NO: 702.

In a sixth aspect, the present invention relates to the use of at least one polynucleotide for detecting at least one miRNA in a biological sample isolated from an individual for determining the risk of the individual to develop Parkinson's disease (PD),
wherein the at least one miRNA is selected from the group consisting of SEQ ID NO: 1 to SEQ ID NO: 702.

In a seventh aspect, the present invention relates to a kit for diagnosing Parkinson's disease (PD) in an individual, for determining the course of Parkinson's disease (PD) in an individual having PD, and/or for determining the risk for developing Parkinson's disease (PD) in an individual comprising:
(i) means for determining the level of at least one miRNA selected from the group consisting of SEQ ID NO: 1 to SEQ ID NO: 702 and a sequence having at least 90% sequence identity thereto in a biological sample isolated from the individual, and
(ii) optionally at least one reference.

This summary of the invention does not necessarily describe all features of the present invention. Other embodiments will become apparent from a review of the ensuing detailed description.

### DETAILED DESCRIPTION OF THE INVENTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention. In the event of a conflict between the definitions or teachings of such incorporated references and definitions or teachings recited in the present specification, the text of the present specification takes precedence.

The term "comprise" or variations such as "comprises" or "comprising" according to the present invention means the inclusion of a stated integer or group of integers but not the exclusion of any other integer or group of integers. The term "consisting essentially of' according to the present invention means the inclusion of a stated integer or group of integers, while excluding modifications or other integers which would materially affect or alter the stated integer. The term "consisting of' or variations such as "consists of' according to the present invention means the inclusion of a stated integer or group of integers and the exclusion of any other integer or group of integers.

The terms "a" and "an" and "the" and similar reference used in the context of describing the invention (especially in the context of the claims) are to be construed to cover both the singular and the plural, unless otherwise indicated herein or clearly contradicted by context.

The terms "microRNA" or "miRNA", as used herein, refer to single-stranded RNA molecules of at least 10 nucleotides and of not more than 45 nucleotides covalently linked together. Preferably, the polynucleotides of the present invention are molecules of 10 to 45 nucleotides or 15 to 35 nucleotides in length, more preferably of 16 to 28 nucleotides or 17 to 27 nucleotides in length, i.e. 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are non-coding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand". This processing is referred to be the canonical miRNA processing pathway.

The terms "microRNA*" or "miRNA*", as used herein, refer to single-stranded RNA molecules of at least 10 nucleotides and of not more than 35 nucleotides covalently linked together. Preferably, the polynucleotides of the present invention are molecules of 10 to 45 nucleotides or 15 to 35 nucleotides in length, more preferably of 16 to 28 nucleotides or 18 to 23 nucleotides in length, i.e. 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used.

Further, the term "miRNA", as used in this context, comprises not only the known miRNAs as e.g. annotated in the miRBase (see next definition) but also other small non-coding RNAs. These are not necessarily processed by the canonical miRNA processing pathway but other enzymes could be involved in maturing the molecules. Specifically, the set of miRNAs contains nucleic acid chains with the same or very similar properties as miRNAs that have been discovered by the inventors from over 2,000 blood data sets containing 100 billion small RNA reads. These can contain nucleic acid chains that are also similar to other non-coding RNA species such as piRNAs. The nucleic acid chains have been detected from the billions of reads by using the software miRMaster that has been recently developed by the inventors.

The term "miRBase", as used herein, refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download.

The term "nucleotides", as used herein, refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar. Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides of the present invention are molecules of 10 to 35 nucleotides or 15 to 45 nucleotides in length, more preferably of 16 to 28 nucleotides or 17 to 27 nucleotides in length, i.e. 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.
In the context of the present invention, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. The polynucleotide variants including polynucleotide fragments or polynucleotide mutants and the miRNA variants including miRNA fragments or miRNA mutants are further defined below. Described herein are polynucleotides in form of single polynucleotide strands as probes for binding to, hybridizing with or detecting complementary sequences of miRNAs (targets), which are selected from the group consisting of SEQ ID NO: 1 to SEQ ID NO: 710, preferably SEQ ID NO: 1 to SEQ ID NO: 702.
The polynucleotide, e.g. the polynucleotide used as a probe for detecting a miRNA or miRNA*, may be unlabeled, directly labeled, or indirectly labeled, such as with biotin to which a streptavidin complex may later bind. The polynucleotide, e.g. the polynucleotide used as a probe for detecting a miRNA or miRNA*, may also be modified, e.g. may comprise an elongation (EL) element. For use in a RAKE or MPEA assay, a polynucleotide with an elongation element may be used as a probe. The elongation element comprises a nucleotide sequence with 1 to 30 nucleotides chosen on the basis of showing low complementarity to potential target sequences, such as nucleotide sequences of miRNAs or miRNAs*, therefore resulting in not to low degree of cross-hybridization to a target mixture. Preferred is a homomeric sequence stretch Nₙ with n = 1 to 30, i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, and N = A or C, or T or G. Particularly preferred is a homomeric sequence stretch Nₙ with n = 1 to 12, i.e. 1, 2, 3, 4, 5, 6, 7, 8, 9, 10, 11, or 12, and N = A or C, or T or G. The polynucleotide, e.g. the polynucleotide used as a probe for detecting a miRNA or miRNA*, may be present in form of a tandem, i.e. in form of a polynucleotide hybrid of two different or identical polynucleotides, both in the same orientation, i.e. 5' to 3' or 3' to 5', or in different orientation, i.e. 5' to 3' and 3' to 5'. Said polynucleotide hybrid/tandem may comprise a spacer element. For use in a tandem hybridization assay, the polynucleotide hybrid/tandem as a probe may comprise a spacer (SP) element. The spacer element represents a nucleotide sequence with n = 0 to 12, i.e. 0, 1, 2, 3, 4, 5, 6, 7, 8, 9, 10, 11, or 12, nucleotides chosen on the basis of showing low complementarity to potential target sequences, such as nucleotide sequences of miRNAs or anti-miRNAs, therefore resulting in not to low degree of cross-hybridization to a target mixture. It is preferred that n is 0, i.e. that there is no spacer between the two miRNA sequence stretches.

The term "differential expression" of a nucleic acid molecule, as used herein, refers to a qualitative and/or quantitative difference in the temporal and/or local nucleic acid molecule expression pattern, e.g. within and/or among biological samples, body fluid samples, cells, or within blood. Thus, a differentially expressed nucleic acid molecule may qualitatively have its expression altered, including an activation or inactivation in, for example, blood from a diseases subject versus blood from a healthy subject. The difference in nucleic acid molecule expression may also be quantitative, e.g. in that expression is modulated, i.e. either up-regulated, resulting in an increased amount of the nucleic acid molecule, or down-regulated, resulting in a decreased amount of the nucleic acid molecule. The degree to which nucleic acid molecule expression differs need only be large enough to be quantified via standard expression characterization techniques, e.g. by quantitative hybridization (e.g. to a microarray, to beads), amplification (PCR, RT-PCR, qRT-PCR, high-throughput RT-PCR), ELISA for quantitation, next generation sequencing (e.g. ABI SOLID, Illumina Genome Analyzer, Roche 454 GS FL), flow cytometry (e.g. LUMINEX) and the like.

The term "label", as used herein, means a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and other entities which can be made detectable. A label may be incorporated into nucleic acids at any position, e.g. at the 3' or 5' end or internally. The polynucleotide for detecting a miRNA (polynucleotide probe) and/or the miRNA itself may be labeled.

The term "stringent hybridization conditions", as used herein, means conditions under which a first nucleic acid sequence (e.g. polynucleotide in its function as a probe for detecting a miRNA or miRNA*) will hybridize to a second nucleic acid sequence (e.g. target sequence such as nucleotide sequence of a miRNA or miRNA*), such as in a complex mixture of nucleic acids. Stringent conditions are sequence-dependent and will be different in different circumstances. Stringent conditions may be selected to be about 5 to 10°C lower than the thermal melting point (Tm) for the specific sequence at a defined ionic strength pH. The Tm may be the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01 to1.0 M sodium ion concentration (or other salts) at pH 7.0 to 8.3 and the temperature is at least about 20°C for short probes (e.g., about 10-35 nucleotides) and up to 60°C for long probes (e.g., greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %,Tween 20, 0.1 x TE buffer, 0.5 mg/ml BSA, 0.1 mg/ml herring sperm DNA, incubating at 42°C with wash in 05x SSPE and 6x SSPE at 45°C.

The term "antisense", as used herein, refers to nucleotide sequences which are complementary to a specific DNA or RNA sequence. The term "antisense strand" is used in reference to a nucleic acid strand that is complementary to the "sense" strand.

Residues in two or more polynucleotide s are said to "correspond" to each other if the residues occupy an analogous position in the polynucleotide structures. It is well known in the art that analogous positions in two or more polynucleotides can be determined by aligning the polynucleotide sequences based on nucleic acid sequence or structural similarities. Such alignment tools are well known to the person skilled in the art and can be, for example, obtained on the World Wide Web, for example, ClustalW (see www.ebi.ac.uk/clustalw) or Align (see http://www.ebi.ac.uk/emboss/align/index.html) using standard settings, preferably for Align EMBOSS::needle, Matrix: Blosum62, Gap Open 10.0, Gap Extend 0.5.

The term "level", as used herein, refers to an amount (measured for example in grams, mole, or counts such as ion or fluorescence counts) or concentration (e.g. absolute or relative concentration) of the miRNA(s) described herein, in particular of the miRNA(s) selected from the group consisting of SEQ ID NO: 1 to SEQ ID NO: 710, preferably SEQ ID NO: 1 to SEQ ID NO: 702.
The term "level", as used herein, also comprises scaled, normalized, or scaled and normalized amounts or values. Preferably, the level determined herein is the expression level.

The term "sensitivity", as used herein, refers to the number of true positive patients (%) with regard to the number of all patients (100%). The individuals may be subjects having Parkinson's disease (PD). The sensitivity is calculated by the following formula: Sensitivity= TP/(TP+FN) (TP= true positives; FN=false negatives).

The term "specificity", as used herein, relates to the number of true negative individuals (%) with regard to the number of all healthy subjects (100%). The specificity is calculated by the following formula: Specificity= TN/(TN+FP) (TN= true negatives; FP=false positives).

The term "accuracy", as used herein, means a statistical measure for the correctness of classification or identification of sample types. The accuracy is the proportion of true results (both true positives and true negatives).

The result of each analysis group is usually calculated from a plurality of isolated samples, i.e. from at least 2 isolated samples, preferably from between 2 and 20, more preferably from between 10 and 60, and even more preferably from between 50 and 100 isolated samples, e.g. selected from the group consisting of subjects not suffering from Parkinson's disease (PD) (i.e. subjects being healthy with respect to PD) and subjects suffering from Parkinson's disease (PD). The methods of the present invention can be carried out in combination with other methods for diagnosing an individual as having/suffering from PD or not or for determining the course of PD in an individual suffering from PD to increase the overall sensitivity and/or specificity. The determination of the level of the miRNA(s) mentioned herein allows the diagnosis of PD in an individual (suspected of having PD) or the determination of the course of PD in an individual suffering from PD.

The term "AUC", as used herein, relates to an abbreviation for the area under a curve. In particular, it refers to the area under a Receiver Operating Characteristic (ROC) curve. The term "Receiver Operating Characteristic (ROC) curve", as used herein, refers to a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve is a measure for the accuracy of a diagnostic test (the larger the area the better, optimum is 1, a random test would have a ROC curve lying on the diagonal with an area of 0.5 (see, for reference, for example, JP. Egan. Signal Detection Theory and ROC Analysis).

The term "Parkinson's disease (PD)", as used herein refers to a group of conditions called motor system disorders, which are the result of the loss of dopamine-producing brain cells. Primary symptoms of PD are tremor, or trembling in hands, arms, legs, jaw, and face; rigidity, or stiffness of the limbs and trunk; bradykinesia, or slowness of movement; and postural instability, or impaired balance and coordination. As these symptoms become more pronounced, patients may have difficulty walking, talking, or completing other simple tasks. PD usually affects people over the age of 50. Early symptoms of PD are subtle and occur gradually. In some people the disease progresses more quickly than in others. There are currently no standard blood or laboratory tests that have been proven to help in diagnosing PD. Therefore, the diagnosis is based on medical history and a neurological examination. PD can be difficult to diagnose accurately. Doctors may sometimes request brain scans or laboratory tests in order to rule out other diseases. At present, there is no cure for PD, but a variety of medications provide dramatic relief from the symptoms. To overcome current roadblocks to better clinical trial design through improved assessment of Parkinson's disease progression across the disease spectrum, there is an urgent unmet need for new diagnostic and progression biomarkers in PD. The present inventions found miRNAs as biomarkers for PD. MiRNAs that are found to be significantly differentially regulated in blood cell preparations derived from a whole blood sample and that are suitable for diagnosing Parkinson's disease are selected from the group consisting of SEQ ID NO: 1 to SEQ ID NO: 710. The miRNA(s) selected from the group consisting of SEQ ID NO: 1 to SEQ ID NO: 702 is (are) particularly preferred.

The term "diagnosing an individual (suspected of having PD) as having Parkinson's disease (PD) or not", as used herein, means determining whether an individual shows signs of or suffers from PD or not. Thus, the individual may be diagnosed as suffering from PD or as not suffering from PD.

The term "determining the course of Parkinson's disease (PD) in an individual having PD", as used herein, means determining the development of PD over time, e.g. whether PD worsens in the individual, does not worsen/is stable in the individual, or improves in the individual over time.

The term "diagnosis", as used herein, refers to the process of determining a possible disease or disorder and, therefore, is a process attempting to define the (clinical) condition of an individual. The determination of the level of at least one miRNA according to the present invention correlates with the (clinical) condition of an individual. Preferably, the diagnosis comprises (i) determining the occurrence/presence of Parkinson's disease, especially in an (very) early phase of the disease, (ii) monitoring the course of Parkinson's disease, (iii) staging of Parkinson's disease, (iv) measuring the response of an individual with Parkinson's disease to therapeutic intervention, and/or (v) segmentation of an individual suffering from Parkinson's disease.

The term "individual", as used herein, refers to any subject for whom it is desired to know whether she or he suffers from/has Parkinson's disease (PD) or not.
Specifically, the term "individual", as used herein, refers to a subject suspected to be affected by PD. The term "individual", as used herein, further refers to a subject having a risk to develop PD/being at risk for the development of PD. The individual may be diagnosed to be affected by PD, i.e. diseased, or may be diagnosed to be not affected by PD, i.e. healthy with respect to PD. The term "individual", as used herein, also refers to a subject that is affected by PD, i.e. diseased. The individual may be retested for PD and may be diagnosed to be still affected by PD, i.e. diseased, or not affected by PD anymore, i.e. healthy with respect to PD, for example after therapeutic intervention. The individual may further be retested for PD and may be diagnosed as having developed an advanced or serious form of PD.
The term "individual" as used herein, also refers to any subject for whom it is desired to know whether she or he may develop PD (in the future). Specifically, the term "individual, as used herein, refers to a subject who has a predisposition to develop PD or will likely develop PD. It may be determined whether the individual has a risk to develop PD/is at risk for the development of PD or not.
It should be noted that an individual that is diagnosed as not suffering from PD, i.e. as being healthy with respect to PD, may possibly suffer from another disease not tested/known.
The individual may be any mammal, including both a human and another mammal, e.g. an animal such as a rabbit, mouse, rat, or monkey. Human subjects as individuals are particularly preferred.

The term "(control) subject", as used herein, refers to a subject known to be not affected by PD (negative control), i.e. healthy with respect to PD. The term "(control) subject", as used herein, also refers to a subject known to be affected by PD, i.e. diseased. Said (control) subject may have developed an advanced form of PD.
It should be noted that a (control) subject which is known as not suffering from PD, i.e. as being healthy with respect to PD, may possibly suffer from another disease not tested/known.
The (control) subject may be any mammal, including both a human and another mammal, e.g. an animal such as a rabbit, mouse, rat, or monkey. Human (control) subjects as individuals are particularly preferred.

The term "treatment", in particular "therapeutic treatment", as used herein, refers to any therapy which improves the health status and/or prolongs (increases) the lifespan of an individual. Said therapy may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease. The disease may be Parkinson's disease (PD). The (therapeutic) treatment of PD includes, but is not limited to, administration of a drug, speech therapy, exercise training, mental training, and/or physical rehabilitation.
The term "biological sample", as used herein, refers to any biological sample from an individual or a (control) subject containing at least one miRNA selected from the group consisting of SEQ ID NO: 1 to SEQ ID NO: 710, preferably SEQ ID NO: 1 to SEQ ID NO: 702.
The biological sample may be a body fluid sample or a tissue sample. For example, biological samples encompassed by the present invention are tissue samples, blood (e.g. whole blood or blood fraction such as blood cell/cellular fraction, serum or plasma) samples, urine samples, cerebrospinal fluid (CSF), or samples from other peripheral sources. Said biological samples may be mixed or pooled, e.g. a sample may be a mixture of a blood sample and a urine sample. Said biological samples may be provided by removing a biological sample from an individual or (control) subject, but may also be provided by using a previously isolated sample. For example, a blood sample may be taken from an individual or (control) subject by conventional blood collection techniques, or a tissue sample may be taken from an individual or (control) subject by biopsy. The biological sample, e.g. urine sample, blood sample or tissue sample, may be obtained from an individual or (control) subject prior to the initiation of a therapeutic treatment, during the therapeutic treatment, and/or after the therapeutic treatment. If the biological sample is obtained from at least one (control) subject, e.g. from at least 1, 2, 3, 4, 5, 6, 7, 8, 9, 10, 20, 30, 40, 50, 60, 70, 80, 90, 100, 150, 200, 250, 300, 400, 500, or 1,000 (control) subject(s), it is designated as "reference biological sample". Preferably, the reference biological sample is from the same source than the biological sample of the individual to be tested, e.g. both are blood samples, urine samples or tissue samples. It is further preferred that both are from the same species, e.g. from a human. It is also (alternatively or additionally) preferred that the measurements of the reference biological sample of the (control) subject and the biological sample of the individual to be tested are identical, e.g. both have an identical volume. It is particularly preferred that the reference biological sample and the biological sample are from (control) subjects/individuals of the same sex and similar age. The term "body fluid sample", as used herein, refers to any liquid sample derived from the body of an individual or (control) subject containing at least one miRNA selected from the group consisting of SEQ ID NO: 1 to SEQ ID NO. 710, preferably SEQ ID NO: 1 to SEQ ID NO: 702. Particularly, the term "body fluid sample", as used herein, refers to any body fluid sample from an individual or (control) subject containing at least one miRNA selected from the group consisting of SEQ ID NO: 1 to SEQ ID NO. 710, preferably SEQ ID NO: 1 to SEQ ID NO: 702.
Said body fluid sample may be a urine sample, blood sample, sputum sample, breast milk sample, cerebrospinal fluid (CSF) sample, cerumen (earwax) sample, gastric juice sample, mucus sample, endolymph fluid sample, perilymph fluid sample, peritoneal fluid sample, pleural fluid sample, saliva sample, sebum (skin oil) sample, semen sample, sweat sample, tears sample, cheek swab, vaginal secretion sample, liquid biopsy, or vomit sample including components or fractions thereof. The term "body fluid sample" also encompasses body fluid fractions, e.g. blood fractions, urine fractions or sputum fractions. The body fluid samples may be mixed or pooled. Thus, a body fluid sample may be a mixture of a blood and a urine sample or a mixture of a blood and cerebrospinal fluid sample. Said body fluid sample may be provided by removing a body liquid from an individual or (control) subject, but may also be provided by using previously isolated body fluid sample material. The body fluid sample allows for a non-invasive analysis of an individual. It is further preferred that the body fluid sample has a volume of between 0.01 and 20 ml, more preferably of between 0.1 and 10 ml, even more preferably of between 0.5 and 8 ml, and most preferably of between 1 and 5 ml. If the body fluid sample is obtained from at least one (control subject), e.g. from at least 1, 2, 3, 4, 5, 6, 7, 8, 9, 10, 20, 30, 40, 50, 60, 70, 80, 90, 100, 150, 200, 250, 300, 400, 500, or 1,000 control subject(s), it is designated as "reference body fluid sample".

The term "blood sample", as used herein, encompasses a whole blood sample or a blood fraction sample such as a blood cell/cellular fraction, blood serum, or blood plasma sample. It is preferred that the blood serum or plasma sample has a volume of between 0.01 and 20 ml, more preferably of between 0.1 and 10 ml, even more preferably of between 0.5 and 8 ml and most preferably of between 1 and 5 ml.
It is preferred that the whole blood sample is collected by means of a blood collection tube. It is, for example, collected in a PAXgene Blood RNA tube, in a Tempus Blood RNA tube, in an EDTA-tube, in a Na-citrate tube, Heparin-tube or in a ACD-tube (Acid citrate dextrose). Preferably, when the whole blood sample is collected the RNA-fraction, especially the miRNA fraction, may be protected/guarded against degradation. For this purpose special collection tubes (e.g. PAXgene Blood RNA tubes from Preanalytix, Tempus Blood RNA tubes from Applied Biosystems) or additives (e.g. RNAlater from Ambion, RNAsin from Promega), that stabilize the RNA fraction and/or the miRNA fraction, may be employed.
It is also preferred that the whole blood sample is collected by means of a bloodspot technique, e.g. using a Mitra Microsampling Device. This technique requires smaller sample volumes, typically 45-60 µl for humans or less. For example, the whole blood may be extracted from the individual via a finger prick with a needle or lancet. Thus, the whole blood sample may have the form of a blood drop. Said blood drop is then placed on an absorbent probe, e.g. a hydrophilic polymeric material such as cellulose, which is capable of absorbing the whole blood. Once sampling is complete, the blood spot is dried in air before transferring or mailing to labs for processing. Because the blood is dried, it is not considered hazardous. Thus no special precautions need be taken in handling or shipping. Once at the analysis site, the desired components, e.g. proteins or metabolites, are extracted from the dried blood spots into a supernatant which is then further analyzed. This technique is suitable for monitoring patients having PD or being at risk for PD at home (on a home care/home sampling basis) or for screening purposes.

The term "blood cell/cellular fraction", as used herein, refers to a blood cell/cellular portion which has been produced from whole blood by removing the extracellular fraction (serum and/or plasma). In other words, the blood cell/cellular fraction is depleted of the extracellular blood components, such as serum and/or plasma. Preferably, the blood cell/cellular portion comprises/essentially consists of/consists of erythrocytes, leukocytes, and/or thrombocytes, e.g. erythrocytes, leukocytes, and thrombocytes.
In one embodiment, the blood sample is a blood cell/cellular fraction. Preferably, the blood cell/cellular fraction comprises/essentially consists of/consists of erythrocytes, leukocytes, and/or thrombocytes, e.g. erythrocytes, leukocytes, and thrombocytes.
In one alternative embodiment, the blood sample is a blood cell sample. Preferably, the blood cell sample comprises/essentially consists of/consists of erythrocytes, leukocytes, and/or thrombocytes, e.g. erythrocytes, leukocytes, and thrombocytes.
In one another alternative embodiment, the blood sample is a blood cell preparation derived from whole blood.

The term "blood cell preparation derived from a whole blood sample", as used herein, refers to a preparation of a whole blood sample that comprises/essentially consists of/consists of blood cells (erythrocytes, leukocytes, and/or thrombocytes, e.g. erythrocytes, leukocytes, and thrombocytes). Preferably, the blood cell preparation does not contain miRNAs that originate from the extra-cellular fraction (e.g. plasma, serum) of whole blood or does contain miRNAs that originate from the extra-cellular fraction (e.g. plasma, serum) only in minor amounts so that these miRNAs do not or do not substantially contribute to the miRNA level in a blood cell preparation derived from a whole blood sample.
Blood cell preparations derived from a whole sample comprising/essentially consisting of/consisting of erythrocytes, leukocytes, and/or thrombocytes, e.g. erythrocytes, leukocytes, and thrombocytes, are obtained from processing of whole blood samples collected in PAXgene Blood RNA Tubes, Tempus Blood RNA Tubes, EDTA-tubes, Na-citrate tubes or Heparin-tubes maintaining or substantially maintaining the initial cellular distribution (blood cell composition) of the whole blood sample. It is preferred that the whole blood sample is collected, e.g. in a PAXgene RNA tube, and processed according to the manufacturers protocol resulting in a blood cell preparation comprising/essentially consisting of/consisting of erythrocytes, leukocytes, and/or thrombocytes, e.g. erythrocytes, leukocytes, and thrombocytes, from which total RNA (comprising the short RNA fraction including the miRNA fraction) is isolated and which is used for determining the miRNA level in said sample according to the present invention.
In another embodiment of the invention the blood cell preparation derived from a whole blood sample comprising/essentially consisting of/consisting of erythrocytes, leukocytes, and/or thrombocytes, e.g. erythrocytes, leukocytes, and thrombocytes, is obtained from processing of a whole blood sample collected in PAXgene Blood RNA Tubes, Tempus Blood RNA Tubes, EDTA-tubes, Na-citrate tubes or Heparin-tubes not necessarily maintaining or not necessarily substantially maintaining the initial cellular distribution (blood cell composition) of the whole blood sample.
With respect to the blood cellular fraction or blood cell preparation comprising/essentially consisting of/consisting of erythrocytes, leukocytes, and thrombocytes, it should be noted that the determined miRNA level represents the (mathematical) average of the levels of said at least one miRNA in the mixture of erythrocytes, leukocytes, and thrombocytes.

The term "total RNA" as used herein relates to the isolated RNA comprising the miRNA-fraction present in a biological sample, e.g. a blood cell preparation derived from a whole blood sample. Preferably, the total RNA according to the present invention contains the miRNA-fraction or contains a miRNA-enriched fraction of the isolated RNA. For example, the total RNA (comprising the miRNA-fraction or miRNA-enriched fraction) is obtained by lysis (e.g. Trizol) of the blood cells in the blood cell preparation, followed by RNA purification e.g. by phenol/chloroform extraction and/or separation based techniques (e.g. glass fiber filter column, silica-membrane column). Examples of kits for RNA isolation and purification include the miRNeasy Kits (Qiagen), PAXgene Blood miRNA Kit (Qiagen), mirVana PARIS Kit (Life Technologies), PARIS Kit (Life Technologies), Tempus Spin RNA Isolation Kit (Life Technologies).

The at least one miRNA referred to herein (SEQ ID NO: 1 to SEQ ID NO: 710) is representative for Parkinson's disease (PD). The term "at least one miRNA representative for Parkinson's disease (PD)", as used herein, refers to at least one fixed defined miRNA which is known to be differential (regulated) between subjects (e.g. humans or other mammals) suffering from Parkinson's disease (PD) (diseased state) and (control) subjects (e.g. humans or other mammals) not suffering from Parkinson's disease (PD) (healthy state with respect to PD) and is, thus, representative for Parkinson's disease. (A) miRNA(s) which is (are) representative for Parkinson's disease (PD)" is (are) selected from the group consisting of SEQ ID NO: 1 to SEQ ID NO: 710, preferably SEQ ID NO: 1 to SEQ ID NO: 702.

In the context of the present invention, the term "kit of parts (in short: kit)" is understood to be any combination of at least some of the components identified herein, which are combined, coexisting spatially, to a functional unit, and which can contain further components. Said kit may allow point-of-care testing (POCT).

The term "point-of-care testing (POCT)", as used herein, refers to a medical diagnostic testing at or near the point of care that is the time and place of individual care. This contrasts with the historical pattern in which testing was wholly or mostly confined to the medical laboratory, which entailed sending off specimens away from the point of care and then waiting hours or days to learn the results, during which time care must continue without the desired information. Point-of-care tests are simple medical tests that can be performed at the bedside. The driving notion behind POCT is to bring the test conveniently and immediately to the individual to be tested. This increases the likelihood that the individual, physician, and care team will receive the results quicker, which allows for immediate clinical management decisions to be made. POCT is often accomplished through the use of transportable, portable, and handheld instruments and test kits. Small bench analyzers or fixed equipment can also be used when a handheld device is not available - the goal is to collect the specimen and obtain the results in a very short period of time at or near the location of the individual so that the treatment plan can be adjusted as necessary before the individual leaves the hospital.

### Embodiments of the invention

There are currently no standard blood or laboratory tests that have been proven to help in diagnosing PD. Therefore, the diagnosis is based on medical history and a neurological examination. PD can be difficult to diagnose accurately. Doctors may sometimes request brain scans or laboratory tests in order to rule out other diseases. At present, there is no cure for PD, but a variety of medications provide dramatic relief from the symptoms. To overcome current roadblocks to better clinical trial design through improved assessment of Parkinson's disease progression across the disease spectrum, there is an urgent unmet need for new diagnostic and progression biomarkers in PD. The present inventions found miRNAs as biomarkers for PD. MiRNAs that are found to be significantly differentially regulated in blood cell preparations derived from a whole blood sample and that are suitable for diagnosing Parkinson's disease are selected from the group consisting of SEQ ID NO: 1 to SEQ ID NO: 710. The miRNA(s) selected from the group consisting of SEQ ID NO: 1 to SEQ ID NO: 702 is (are) particularly preferred.

Thus, in a first aspect, the present invention relates to a method for diagnosing Parkinson's disease (PD) in an individual (suspected of having PD) comprising the step of:
determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual (suspected of having PD).

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In one embodiment, the level of the at least one miRNA is compared to a reference level of said at least one miRNA. Thus, in one particular embodiment, the present invention relates to a method for diagnosing Parkinson's disease (PD) in an individual (suspected of having PD) comprising the steps of
(i) determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual (suspected of having PD), and
(ii) comparing the level of the at least one miRNA to a reference level of said at least one miRNA.
The above comparison allows to determine whether an individual has/suffers from PD or not.

The reference level may be any level which allows to determine whether an individual suffers from PD or not. It may be obtained from a (control) subject (i.e. a subject different from the individual to be tested/diagnosed) or from the same individual. In the latter case, the individual may be retested for PD, e.g. in the form of a longitudinal monitoring. It may be determined that the individual is now affected by PD or still not affected by PD.

In one preferred embodiment, the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), isolated from at least one (control) subject not suffering from PD, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 (control) subject(s) not suffering from PD. The at least one subject not suffering from PD can be considered as being healthy with respect to PD. It is more preferred that the reference level is the level determined by measuring between 2 and 500 reference biological samples isolated from between 2 and 500 subjects not suffering from PD. It is even more preferred that the reference level is determined by measuring between 50 and 500 reference biological samples isolated from between 50 and 500 subjects not suffering from PD. It is most preferred that the reference level is determined by measuring between 100 and 500 reference biological samples isolated from between 100 and 500 subjects not suffering from PD.

It is practicable to take one reference biological sample per subject for analysis. If additional reference biological samples are required, e.g. to determine the reference level in different reference biological samples, the same subject may be (re)tested. Said reference level may be an average reference level. It may be determined by measuring reference levels and calculating the "average" value (e.g. mean, median or modal value) thereof. It is preferred that the reference biological sample is from the same source (e.g. blood sample) than the biological sample isolated from the individual. It is further preferred that the reference level is obtained from a subject of the same gender (e.g. female or male) and/or of a similar age/phase of life (e.g. adults or elderly) than the individual to be tested or diagnosed.

As mentioned above, the level of the at least one miRNA is compared to a reference level of said at least one miRNA. Said reference level is the level determined by measuring a reference biological sample. For example, if the level of the miRNA according to SEQ ID NO: 1 is determined in a biological sample isolated from an individual, it is compared to a reference level of the miRNA according to SEQ ID NO: 1 determined in a reference biological sample. Alternatively, if the level of the miRNA according to SEQ ID NO: 1 and the level of the miRNA according to SEQ ID NO: 2 is determined in a biological sample isolated from an individual, both levels are compared to the respective reference levels, i.e. the level of the miRNA according to SEQ ID NO: 1 is compared to the reference level of the miRNA according to SEQ ID NO: 1 and the level of the miRNA according to SEQ ID NO: 2 is compared to the reference level of the miRNA according to SEQ ID NO: 2 determined in a reference biological sample.

In one more preferred embodiment,
(a) the level of the at least one miRNA selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299, 301, 305, 310, 311, 312, 317, 319, 320, 321, 323, 324, 326, 332, 336, 339, 341, 342, 343, 344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90%, preferably at least 95%, more preferably at least 99%, i.e. at least 90, 91, 92, 93, 94, 95, 96, 97, 98, or 99%, sequence identity thereto is below the reference level, which indicates that the individual has PD, and/or
(b) the level of the at least one miRNA selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90%, preferably at least 95%, more preferably at least 99%, i.e. at least 90, 91, 92, 93, 94, 95, 96, 97, 98, or 99%, sequence identity thereto is above the reference level, which indicates that the individual has PD.

In particular, the level of the at least one miRNA is at least 0.4-fold, at least 0.5-fold, at least 0.6-fold or at least 0.7-fold, preferably at least 0.8-fold or at least 0.9-fold, more preferably at least 1.2-fold or at least 1.5-fold, and even more preferably at least 2.0-fold or at least 3.0-fold below/above the reference level. For example, the level of the at least one miRNA is at least 0.4-fold, at least 0.5-fold, at least 0.6-fold, at least 0.7-fold, at least 0.8-fold, at least 0.9-fold, at least 1.0-fold, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

In addition to the level of the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, the level of at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably determined.

In a second aspect, the present invention relates to a method for determining the course of Parkinson's disease (PD) in an individual having PD comprising the step of:
determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual (having PD).

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In one embodiment, the level of the at least one miRNA is compared to a reference level of said at least one miRNA. Thus, in one particular embodiment, the present invention relates to a method for determining the course of Parkinson's disease (PD) in an individual having PD comprising the steps of
(i) determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual (having PD), and
(ii) comparing the level of the at least one miRNA to a reference level of said at least one miRNA.
The above comparison allows to determine the course of PD in the individual having PD. It may be determined that PD worsens in the individual, that PD does not worsen/is stable in the individual, or that PPMS improves in the individual.

The reference level may be any level which allows to determine the course of PD. It may be obtained from a (control) subject (i.e. a subject different from the individual to be tested/analyzed) or from the same individual.

In one preferred embodiment, the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), isolated from at least one (control) subject not suffering from PD, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 (control) subject(s) not suffering from PD. The at least one subject not suffering from PD can be considered as being healthy with respect to PD. It is more preferred that the reference level is the level determined by measuring between 2 and 500 reference biological samples isolated from between 2 and 500 subjects not suffering from PD. It is even more preferred that the reference level is determined by measuring between 50 and 500 reference biological samples isolated from between 50 and 500 subjects not suffering from PD. It is most preferred that the reference level is determined by measuring between 100 and 500 reference biological samples isolated from between 100 and 500 subjects not suffering from PD.

In one another preferred embodiment, the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), isolated from at least one (control) subject suffering from PD, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 (control) subject(s) suffering from PD. It is more preferred that the reference level is the level determined by measuring between 2 and 500 reference biological samples isolated from between 2 and 500 subjects suffering from PD. It is even more preferred that the reference level is determined by measuring between 50 and 500 reference biological samples isolated from between 50 and 500 subjects suffering from PD. It is most preferred that the reference level is determined by measuring between 100 and 500 reference biological samples isolated from between 100 and 500 subjects suffering from PD.

It is practicable to take one reference biological sample per subject for analysis. If additional reference biological samples are required, e.g. to determine the reference level in different reference biological samples, the same subject may be (re)tested. Said reference level may be an average reference level. It may be determined by measuring reference levels and calculating the "average" value (e.g. mean, median or modal value) thereof. It is preferred that the reference biological sample is from the same source (e.g. blood sample) than the biological sample isolated from the individual. It is further preferred that the reference level is obtained from a subject of the same gender (e.g. female or male) and/or of a similar age/phase of life (e.g. adults or elderly) than the individual to be tested or analysed.

In one alternative or additional preferred embodiment, said determining comprises determining the level of the at least one miRNA in a biological sample at a first point in time and in at least one further biological sample at a later point in time and comparing said levels determined at the different time points. The above analysis is performed on the same individual. Thus, in one particular embodiment, the present invention relates to a method for determining the course of Parkinson's disease (PD) in an individual having PD comprising the steps of
(i) determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample from an individual having PD at a first point in time and in at least one further biological sample from the (same) individual having PD at a later point in time, and
(ii) comparing said levels determined at the different time points. In one more preferred embodiment,
   (a) the at least one miRNA is selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299, 301, 305, 310, 311, 312, 317, 319, 320, 321, 323, 324, 326, 332, 336, 339, 341, 342, 343, 344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90%, preferably at least 95%, more preferably at least 99%, i.e. at least 90, 91, 92, 93, 94, 95, 96, 97, 98, or 99%, sequence identity thereto and wherein the level of said at least one miRNA which
      (i) decreases over time indicates that PD worsens in the individual,
      (ii) does not change over time indicates that PD does not worsen/is stable in the individual, or
      (iii) increases over time indicates that PD improves in the individual, and/or
   (b) the at least one miRNA is selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90%, preferably at least 95%, more preferably at least 99%, i.e. at least 90, 91, 92, 93, 94, 95, 96, 97, 98, or 99%, sequence identity thereto and wherein the level of said at least one miRNA which
      (i) increases over time indicates that PD worsens in the individual,
      (ii) does not change over time indicates that PD does not worsen/is stable in the individual, or
      (iii) decreases over time indicates that PD improves in the individual.

As mentioned above, the detection of a decrease/an increase (dependent on the miRNA detected) of the level over time indicates that PD worsens in the individual. Preferably, said decrease/increase is at least 0.4-fold, at least 0.5-fold, at least 0.6-fold or at least 0.7-fold over time. More preferably, said decrease/increase is at least 0.8-fold or at least 0.9-fold over time. Even more preferably, said decrease/increase is at least 1.2-fold or at least 1.5-fold over time. Most preferably, said decrease/increase is at least 2.0-fold or at least 3.0-fold over time. For example, said decrease/increase may be determined over 1 year (12 months) or over 2 years (24 months).

As mentioned above, a level which does not change over time indicates that PD does not worsen/is stable in the individual. "Does not change over time" in this respect may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Does not change over time" in this respect may also mean that the detected level variation is within the accuracy of a measurement. The accuracy of a measurement depends on the measurement method used. Preferably, the level is constant over time.

As mentioned above, the detection of an increase/a decrease (dependent on the miRNA detected) of the level over time indicates that PD improves in the individual. Preferably, said increase/decrease is at least 0.4-fold, at least 0.5-fold, at least 0.6-fold or at least 0.7-fold over time. More preferably, said increase/decrease is at least 0.8-fold or at least 0.9-fold over time. Even more preferably, said increase/decrease is at least 1.2-fold or at least 1.5-fold over time. Most preferably, said increase/decrease is at least 2.0-fold or at least 3.0-fold over time. For example, said increase/decrease may be determined over 1 year (12 months) or over 2 years (24 months).

The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the individual may be routinely checked, e.g. once or twice a year. The individual may be (re)tested at 2, 3, 4, 5, 6 7, 8, 9, or 10 time points (first point in time and further point(s) in time).

In addition to the determination of the course of PD, the treatment of this disease can be monitored. It is namely preferred that the individual receives or has received a treatment, in particular therapeutic treatment, of PD during the determination of the course of PD. The treatment of PD may be selected from the group consisting of the administration of a drug, speech therapy, exercise training, mental training, and physical rehabilitation.

In one even more preferred embodiment,
(a) the at least one miRNA is selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299, 301, 305, 310, 311, 312, 317, 319, 320, 321, 323, 324, 326, 332, 336, 339, 341, 342, 343, 344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90%, preferably at least 95%, more preferably at least 99%, i.e. at least 90, 91, 92, 93, 94, 95, 96, 97, 98, or 99%, sequence identity thereto, wherein the individual receives or has received a treatment for PD, and wherein the level of said at least one miRNA which
   (i) increase over time indicates that the individual responds to said treatment,
   (ii) does not change over time indicates that the individual does not respond to said treatment or that PD is stable in the individual due to said treatment, or
   (iii) decreases over time indicates that the individual does not respond to said treatment, and/or
(b) the at least one miRNA is selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90%, preferably at least 95%, more preferably at least 99%, i.e. at least 90, 91, 92, 93, 94, 95, 96, 97, 98, or 99%, sequence identity thereto, wherein the individual receives or has received a treatment for PD, and wherein the level of said at least one miRNA which
   (i) decreases over time indicates that the individual responds to said treatment,
   (ii) does not change over time indicates that the individual does not respond to said treatment or that PD is stable in the individual due to said treatment, or
   (iii) increases over time indicates that the individual does not respond to said treatment.

As mentioned above, the detection of an increase/a decrease (dependent on the miRNA detected) of the level over time indicates that the individual responds to said treatment. Preferably, said increase/decrease is at least 0.4-fold, at least 0.5-fold, at least 0.6-fold or at least 0.7-fold over time. More preferably, said increase/decrease is at least 0.8-fold or at least 0.9-fold overtime. Even more preferably, said increase/decrease is at least 1.2-fold or at least 1.5-fold over time. Most preferably, said increase/decrease is at least 2.0-fold or at least 3.0-fold over time. For example, said increase/decrease may be determined over 1 year (12 months) or over 2 years (24 months).

As mentioned above, a level which does not change over time indicates that the individual does not respond to said treatment or that PD is stable in the individual due to said treatment. "Does not change over time" in this respect may mean that the level varies over time between 0 and < 20%, e.g. 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 19.9, 19.99, or 19.999%. "Does not change over time" in this respect may also mean that the detected level variation is within the accuracy of a measurement. The accuracy of a measurement depends on the measurement method used. Preferably, the level is constant over time.

As mentioned above, the detection of a decrease/an increase (dependent on the miRNA detected) of the level over time indicates that the individual does not respond to said treatment. Preferably, said decrease/increase is at least 0.4-fold, at least 0.5-fold, at least 0.6-fold or at least 0.7-fold overtime. More preferably, said decrease/increase is at least 0.8-fold or at least 0.9-fold over time. Even more preferably, said decrease/increase is at least 1.2-fold or at least 1.5-fold over time. Most preferably, said decrease/increase is at least 2.0-fold or at least 3.0-fold over time. For example, said decrease/increase may be determined over 1 year (12 months) or over 2 years (24 months).

The individual may receive a treatment during the complete determination/monitoring process (e.g. the administration of a drug) or may receive a treatment before, at, or after a first point in time (e.g. the administration of a drug) and may be retested at a later point in time. In particular, said first point in time may be before the initiation of a treatment and said later point in time may be during the treatment and/or after the treatment. If the treatment encompasses the administration of a drug and the individual responds to said treatment, the drug administration may be continued, the dose of the drug may be reduced, or the drug administration may be stopped. If the treatment encompasses the administration of a drug and the individual does not respond to said treatment, the dose of the drug may be increased, the drug may be changed, or the therapy mode may be changed, e.g. from drug administration to exercise training, mental training speech therapy, and/or physical rehabilitation.

In addition to the level of the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, the level of at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably determined.

In a third aspect, the present invention relates to a method for determining the risk for developing Parkinson's disease (PD) in an individual comprising the step of:
determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In one embodiment, the level of the at least one miRNA is compared to a reference level of said at least one miRNA. Thus, in one particular embodiment, the present invention relates to a method for determining the risk for developing Parkinson's disease (PD) in an individual comprising the steps of:
(i) determining the level of at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual (suspected of having PD), and
(ii) comparing the level of the at least one miRNA to a reference level of said at least one miRNA.
The above comparison allows to determine whether the patient is at risk for the development of PD or not. The above method may also be designated as a screening method. Said screening method allows an early detection of PD and, thus, the identification of individuals having a predisposition or a likelihood (e.g. on the basis of their physical and/or physiological conditions) to develop PD, e.g. in the near future, such as within the next 1 or 2 years.

The reference level may be any level which allows to determine whether an individual is at risk for developing PD or not. It may be obtained from a (control) subject (i.e. a subject different from the individual to be tested/analyzed) or from the same individual. In the latter case, the individual may be retested for the risk to develop PD. It may be determined that the individual has now a risk to develop PD or still not.

In one preferred embodiment, the reference level is the level determined by measuring at least one reference biological sample, e.g. at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 reference biological sample(s), isolated from at least one (control) subject not suffering from PD, e.g. from at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 150, 200, 250, 300, 400, 500, or 1.000 (control) subject(s) not suffering from PD. The at least one subject not suffering from PD can be considered as being healthy with respect to PD. It is more preferred that the reference level is the level determined by measuring between 2 and 500 reference biological samples isolated from between 2 and 500 subjects not suffering from PD. It is even more preferred that the reference level is determined by measuring between 50 and 500 reference biological samples isolated from between 50 and 500 subjects not suffering from PD. It is most preferred that the reference level is determined by measuring between 100 and 500 reference biological samples isolated from between 100 and 500 subjects not suffering from PD.

It is practicable to take one reference biological sample per subject for analysis. If additional reference biological samples are required, e.g. to determine the reference level in different reference biological samples, the same subject may be (re)tested. Said reference level may be an average reference level. It may be determined by measuring reference levels and calculating the "average" value (e.g. mean, median or modal value) thereof. It is preferred that the reference biological sample is from the same source (e.g. blood sample) than the biological sample isolated from the individual. It is further preferred that the reference level is obtained from a subject of the same gender (e.g. female or male) and/or of a similar age/phase of life (e.g. adults or elderly) than the individual to be tested or analysed.

In one more preferred embodiment,
(a) the level of the at least one miRNA selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299, 301, 305, 310, 311, 312, 317, 319, 320, 321, 323, 324, 326, 332, 336, 339, 341, 342, 343, 344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90%, preferably at least 95%, more preferably at least 99%, i.e. at least 90, 91, 92, 93, 94, 95, 96, 97, 98, or 99%, sequence identity thereto is below the reference level, which indicates that the individual is at risk for developing PD, and/or
(b) the level of the at least one miRNA selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90%, preferably at least 95%, more preferably at least 99%, i.e. at least 90, 91, 92, 93, 94, 95, 96, 97, 98, or 99%, sequence identity thereto is above the reference level, which indicates that the individual is at risk for developing PD.

In particular, the level of the at least one miRNA is at least 0.4-fold, at least 0.5-fold, at least 0.6-fold or at least 0.7-fold, preferably at least 0.8-fold or at least 0.9-fold, more preferably at least 1.2-fold or at least 1.5-fold, and even more preferably at least 2.0-fold or at least 3.0-fold below/above the reference level. For example, the level of the at least one miRNA is at least 0.4-fold, at least 0.5-fold, at least 0.6-fold, at least 0.7-fold, at least 0.8-fold, at least 0.9-fold, at least 1.0-fold, at least 1. 1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 2.1-fold, at least 2.2-fold, at least 2.3-fold, at least 2.4-fold, at least 2.5-fold, at least 2.6-fold, at least 2.7-fold, at least 2.8-fold, at least 2.9-fold, or at least 3.0-fold below/above the reference level.

In addition to the level of the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, the level of at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably determined.

In the methods of the first to third aspect of the present invention, it is preferred that the individual is a mammal, preferably a human.

In the methods of the first to third aspect of the present invention, it is further preferred that the biological sample is a body fluid sample or a tissue sample. Preferably, the body fluid sample is a blood sample. More preferably, the blood sample is a whole blood sample or a blood fraction sample. Even more preferably, the blood fraction sample is a blood cell/cellular fraction sample, a blood serum sample, or a blood plasma sample. Most preferably, the blood fraction sample is a blood cell/cellular fraction sample.
In one embodiment, the blood cell/cellular fraction comprises/essentially consists of/consists of erythrocytes, leukocytes, and/or thrombocytes, e.g. erythrocytes, leukocytes, and thrombocytes.

If in the context of the first to third aspect of the present invention, the level of more than one miRNA is determined, e.g. of two or more miRNAs, it is referred herein to a set comprising at least two miRNAs.

The determination of the level of the at least one miRNA may be carried out by any convenient means for determining the level of a nucleotide sequence such as miRNA. For this purpose, qualitative, semi-quantitative and quantitative detection methods can be used. Quantitative detection methods are preferred. A variety of techniques are well known to the person skilled in the art. For example, the level of the at least one miRNA can be determined in the methods of the first to third aspect of the present invention by nucleic acid hybridization, nucleic acid amplification, polymerase extension, sequencing, mass spectroscopy, an immunochemical method, or any combination thereof.

Preferably,
(i) the nucleic acid hybridization is performed using a microarray/biochip, or using *in situ* hybridization,
(ii) the nucleic acid amplification is performed using real-time PCR (RT-PCR) or quantitative real-time PCR (qPCR),
(iii) the sequencing is next generation sequencing, or
(iv) the immunochemical method is an enzyme linked immunosorbent assay (ELISA).

Nucleic acid amplification, for example, may be performed using real time polymerase chain reaction (RT-PCR) such as real time quantitative PCR (RT qPCR). The real time polymerase chain reaction (RT-PCR) may include the following steps: (i) extracting total RNA from the biological sample isolated from the individual, (ii) obtaining cDNA samples by RNA reverse transcription (RT) reaction using miRNA-specific primers, (iii) designing miRNA-specific cDNA forward primers and providing universal reverse primers to amplify the cDNA via polymerase chain reaction (PCR), (iv) adding a fluorescent probe to conduct PCR, and (v) detecting and comparing the variation in levels of miRNAs in the biological sample isolated from the individual relative to those of miRNAs in a reference biological sample isolated from a (control) subject.
A variety of kits and protocols to determine the miRNA level by real time polymerase chain reaction (RT-PCR) such as real time quantitative PCR (RT qPCR) are available. For example, reverse transcription of miRNAs may be performed using the TaqMan MicroRNA Reverse Transcription Kit (Applied Biosystems) according to manufacturer's recommendations.

Nucleic acid hybridization, for example, may be performed using a microarray/biochip or *in situ* hybridization. For nucleic acid hybridization, for example, the polynucleotides (probes) described herein with complementarity to the corresponding miRNAs to be detected are attached to a solid phase to generate a microarray/biochip. Said microarray/biochip is then incubated with miRNAs, isolated (e.g. extracted) from the biological sample, which may be labelled or unlabelled. Upon hybridization of the labelled miRNAs to the complementary polynucleotide sequences on the microarray/biochip, the success of hybridisation may be controlled and the intensity of hybridization may be determined via the hybridisation signal of the label in order to determine the level of each tested miRNA in said biological sample.

Alternatively, the miRNA level may be determined using an immunochemical method, e.g. using an ELISA. Said method may include the following steps: (i) isolating miRNAs from a biological sample, (ii) hybridizing polynucleotide probes (complementary) to the miRNAs to obtain hybrids of said polynucleotides probes and said miRNAs, and (iii) binding said hybrids to antibodies capable of specifically binding hybrids of said polynucleotide probes and said miRNAs, and (iv) detecting the antibody-bound hybrids.

In the methods of the first to third aspect of the present invention, it is further preferred that the level of the at least one miRNA is the expression level of said at least one miRNA.

The methods of the first to third aspect of the present invention are *in vitro* methods.

In a fourth aspect, the present invention relates to the use of at least one polynucleotide (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 polynucleotide(s)) for detecting at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) in a biological sample isolated from an individual (suspected of having PD) for diagnosing Parkinson's disease (PD) in the individual (suspected of having PD),
wherein the at least one miRNA is selected from the group consisting of SEQ ID NO: 1 to SEQ ID NO: 702.

The at least one polynucleotide may be a probe/primer, in particular a primer pair.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In one preferred embodiment,
(i) the at least one polynucleotide is at least partially (reverse) complementary, preferably (reverse) complementary, to the at least one miRNA mentioned above, or
(ii) the at least one polynucleotide has at least 90%, preferably at least 95%, more preferably at least 99%, i.e. at least 90, 91, 92, 93, 94, 95, 96, 97, 98, or 99%, sequence identity to the polynucleotide according to (i).
It is particularly preferred that the polynucleotide as defined in (ii) has at least 90%, preferably at least 95%, more preferably at least 99%, i.e. at least 90, 91, 92, 93, 94, 95, 96, 97, 98, or 99%, sequence identity over a continuous stretch of at least 10, 11, 12, 13, 14, 15, 16, 17, 18, 19, 20, 21, 22, 23, 24, 25, or more nucleotides, preferably over the whole length, to the polynucleotide according to (i).
In addition, the polynucleotide as defined in (ii) (i.e. polynucleotide variant) is only regarded as a polynucleotide as defined in (ii) (i.e. polynucleotide variant) within the context of the present invention, if it is still capable of binding to, hybridizing with, or detecting the respective target nucleic acid molecule, i.e. the target nucleic acid molecule comprising a nucleotide sequence selected from the group consisting of SEQ ID NO: 1 to SEQ ID NO: 702, through one or more types of chemical bonds, usually through complementary base pairing, usually through hydrogen bond formation under stringent hybridization conditions. The skilled person can readily assess whether a polynucleotide as defined in (ii) (i.e. polynucleotide variant) is still capable of binding to, hybridizing with, recognizing or detecting the respective target nucleic acid molecule, i.e. the target nucleic acid molecule comprising a nucleotide sequence selected from the group consisting of SEQ ID NO: 1 to SEQ ID NO: 702. Suitable assays to determine whether hybridization under stringent conditions still occurs are well known in the art. However, as an example, a suitable assay to determine whether hybridization still occurs comprises the steps of: (a) incubating the polynucleotide as defined in (ii) or (iii) attached onto a biochip with the respective target nucleic acid molecule, i.e. the target nucleic acid molecule comprising a nucleotide sequence selected from the group consisting of SEQ ID NO: 1 to SEQ ID NO: 702, (b) washing the biochip to remove unspecific bindings, (c) subjecting the biochip to a detection system, and (d) analyzing whether the polynucleotide can still hybridize with the respective target nucleic acid molecule. As a positive control, the respective non-mutated polynucleotide as defined in (i) may be used. Preferably stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %,Tween 20, 0.1 x TE buffer, 0.5 mg/ml BSA, 0.1 mg/ml herring sperm DNA, incubating at 42°C with wash in 05x SSPE and 6x SSPE at 45°C.

The at least one polynucleotide (probe/primer, in particular primer pair) described above is useful for conducting the method according to the first aspect of the present invention.

In addition to the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably detected.

In a fifth aspect, the present invention relates to the use of at least one polynucleotide (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 polynucleotide(s)) for detecting at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) in a biological sample isolated from an individual having Parkinson's disease (PD) for determining the course of PD in the individual (having PD), wherein the at least one miRNA is selected from the group consisting of SEQ ID NO: 1 to SEQ ID NO: 702.

The at least one polynucleotide may be a probe/primer, in particular a primer pair.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In one preferred embodiment,
(i) the at least one polynucleotide is at least partially (reverse) complementary, preferably (reverse) complementary, to the at least one miRNA mentioned above, or
(ii) the at least one polynucleotide has at least 90%, preferably at least 95%, more preferably at least 99%, i.e. at least 90, 91, 92, 93, 94, 95, 96, 97, 98, or 99%, sequence identity to the polynucleotide according to (i).
It is particularly preferred that the polynucleotide as defined in (ii) has at least 90%, preferably at least 95%, more preferably at least 99%, i.e. at least 90, 91, 92, 93, 94, 95, 96, 97, 98, or 99%, sequence identity over a continuous stretch of at least 10, 11, 12, 13, 14, 15, 16, 17, 18, 19, 20, 21, 22, 23, 24, 25, or more nucleotides, preferably over the whole length, to the polynucleotide according to (i).

As to the polynucleotide variants, it is referred to the fourth aspect of the present invention.

The at least one polynucleotide (probe/primer, in particular primer pair) described above is useful for conducting the method according to the second aspect of the present invention.

In addition to the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably detected.

In a sixth aspect, the present invention relates to the use of at least one polynucleotide (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 polynucleotide(s)) for detecting at least one miRNA (e.g. 1, 2, 3, 4, 5, 6, 7, 8, 9, 10, 11, 12, 13, 14, 15, 16, 17, 18, 19, 20, 21, 22, 23, 24, 25, 50, 100, 150, 200, 300, 400, 500, 600, 700, or 702 miRNA(s)) in a biological sample isolated from an individual for determining the risk of the individual to develop Parkinson's disease (PD),
wherein the at least one miRNA is selected from the group consisting of SEQ ID NO: 1 to SEQ ID NO: 702.

The at least one polynucleotide may be a probe/primer, in particular a primer pair.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In one preferred embodiment,
(i) the at least one polynucleotide is at least partially (reverse) complementary, preferably (reverse) complementary, to the at least one miRNA mentioned above, or
(ii) the at least one polynucleotide has at least 90%, preferably at least 95%, more preferably at least 99%, i.e. at least 90, 91, 92, 93, 94, 95, 96, 97, 98, or 99%, sequence identity to the polynucleotide according to (i).
It is particularly preferred that the polynucleotide as defined in (ii) has at least 90%, preferably at least 95%, more preferably at least 99%, i.e. at least 90, 91, 92, 93, 94, 95, 96, 97, 98, or 99%, sequence identity over a continuous stretch of at least 10, 11, 12, 13, 14, 15, 16, 17, 18, 19, 20, 21, 22, 23, 24, 25, or more nucleotides, preferably over the whole length, to the polynucleotide according to (i).

As to the polynucleotide variants, it is referred to the fourth aspect of the present invention.

The at least one polynucleotide (probe/primer, in particular primer pair) described above is useful for conducting the method according to the third aspect of the present invention.

In addition to the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably detected.

In the use of the fourth to sixth aspect of the present invention, it is preferred that the individual is a mammal, preferably a human.

In the use of the fourth to sixth aspect of the present invention, it is further preferred that the biological sample is a body fluid sample or a tissue sample. Preferably, the body fluid sample is a blood sample. More preferably, the blood sample is a whole blood sample or a blood fraction sample. Even more preferably, the blood fraction sample is a blood cell/cellular fraction sample, a blood serum sample, or a blood plasma sample. Most preferably, the blood fraction sample is a blood cell/cellular fraction sample.
In one embodiment, the blood cell/cellular fraction comprises/essentially consists of/consists of erythrocytes, leukocytes, and/or thrombocytes, e.g. erythrocytes, leukocytes, and thrombocytes.

If in the context of the fourth to sixth aspect of the present invention, more than one miRNA is detected, e.g. two or more miRNAs, it is referred herein to a set comprising at least two miRNAs.

In a seventh aspect, the present invention relates to (the use of) a kit for diagnosing Parkinson's disease (PD) in an individual (suspected of having PD), for determining the course of Parkinson's disease (PD) in an individual (having PD), and/or for determining the risk for developing Parkinson's disease (PD) in an individual comprising:
(i) means for determining the level of at least one miRNA selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto in a biological sample isolated from the individual, and
(ii) optionally at least one reference.

In particular, the kit is useful for carrying out the methods of the first, second, and/or third aspect of the present invention.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

The at least one reference may be any reference which allows to diagnose whether an individual (suspected of having PD) suffers from PD or not, to determine the course of Parkinson's disease (PD) in an individual (having PD), and/or to determine the risk for developing Parkinson's disease (PD) in an individual. In this respect, it is also referred to the preferred embodiments mentioned in the context of the first, second, and/or third aspect of the present invention.

In particular, the means in (i) comprise
at least one polynucleotide (probe), in particular according to the fourth to sixth aspect of the present invention,
at least one primer pair, in particular according to the fourth to sixth aspect of the present invention, and/or
at least one polynucleotide (probe), in particular according to the fourth to sixth aspect of the present invention, and at least one antibody capable of binding a hybrid of said at least one polynucleotide (probe) and said at least one miRNA.

Said means allow to determine the level of the at least one miRNA in a biological sample isolated from an individual and, thus, to diagnose whether the individual (suspected of having PD) suffers from PD or not, to determine the course of Parkinson's disease (PD) in an individual (having PD), and/or to determine whether the individual is at risk for developing Parkinson's disease (PD) or not.

The at least one polynucleotide (probe) may be part of a microarray/biochip or may be attached to beads of a beads-based multiplex system.

The at least one polynucleotide (primer, primer pair) may be part of a RT-PCR system, a PCR-system, or a next generation sequencing system.

Said means may further comprise a microarray, a RT-PCT system, a PCR-system, a flow cytometer, a Luminex system and/or a next generation sequencing system.

The kit may further comprise
(iii) a container, and/or
(iv) a data carrier.
The data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. The access code may also allow access to an application software that causes a computer to perform tasks for computer users or a mobile app which is a software designed to run on smartphones and other mobile devices.
Said data carrier may further comprise the at least one reference, e.g. the reference level of the level of the at least one miRNA determined herein. In case that the data carrier comprises an access code which allows the access to a database, said at least one reference, e.g. said reference level may be deposited in this database.
The data carrier may also comprise information or instructions on how to carry out the methods according to the first to third aspect of the present invention.

Said kit may also comprise materials desirable from a commercial and user standpoint including a buffer(s), a reagent(s) and/or a diluent(s) for determining the level mentioned above.

In addition to the level of the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, the level of at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably determined.

In a further aspect, the present invention relates to the use of at least one miRNA isolated from a biological sample from an individual for diagnosing Parkinson's disease (PD),
wherein the at least one miRNA is selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In addition to the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably used.

As to the biological sample and/or the individual, it is referred to first aspect and/or to the definition part of the present invention.

In another further aspect, the present invention relates to the use of at least one miRNA isolated from a biological sample from an individual having Parkinson's disease (PD) for determining the course of PD in the individual,
wherein the at least one miRNA is selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In addition to the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably used.

As to the biological sample and/or the individual, it is referred to second aspect and/or to the definition part of the present invention.

In another further aspect, the present invention relates to the use of at least one miRNA isolated from a biological sample from an individual for determining the risk of the individual to develop Parkinson's disease (PD),
wherein the at least one miRNA is selected from the group consisting of SEQ ID NO: 1 to SEQ ID NO: 702 and a sequence having at least 90%, preferably at least 95%, more preferably at least 99%, i.e. at least 90, 91, 92, 93, 94, 95, 96, 97, 98, or 99%, sequence identity thereto.

Preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, or 119 miRNA(s)) is selected from the group consisting of SEQ ID NOs: 5, 25, 26, 35, 38, 41, 43, 44, 45, 46, 47, 48, 49, 50, 56, 57, 58, 62, 65, 68, 70, 71, 74, 75, 76, 77, 79, 80, 81, 84, 98, 100, 107, 126, 130, 137, 139, 145, 147, 148, 150, 165, 168, 171, 173, 181, 188, 200, 201, 209, 212, 218, 219, 220, 224, 230, 243, 257, 258, 261, 273, 280, 281, 286, 296, 306, 308, 314, 318, 330, 331, 335, 368, 425, 432, 435, 480, 481, 507, 536, 550, 569, 572, 623, 627, 633, 636, 637, 638, 640, 641, 643, 646, 647, 650, 651, 654, 655, 656, 657, 661, 663, 667, 668, 669, 673, 677, 681, 682, 684, 685, 687, 688, 689, 695, 696, 698, 701, 702, and a sequence having at least 90%, preferably at least 95%, more preferably at least 99%, i.e. at least 90, 91, 92, 93, 94, 95, 96, 97, 98, or 99%, sequence identity thereto.

More preferably, the at least one miRNA (e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 miRNA(s)) is selected from the group consisting of SEQ ID NOs: 5, 25, 26, 35, 38, 41, 43, 44, 45, 47, 49, 50, 58, 62, 65, 68, 70, 80, 81, 84, 98, 100, 107, 126, 130, 137, 139, 145, 147, 148, 150, 165, 168, 171, 173, 181, 188, 200, 201, 212, 218, 286, 314, 550, 641, 654, 663, 667, 673, 689, and a sequence having at least 90%, preferably at least 95%, more preferably at least 99%, i.e. at least 90, 91, 92, 93, 94, 95, 96, 97, 98, or 99%, sequence identity thereto.

Even more preferably, the at least one miRNA (e.g. 1, 2, 3, 4, 5, 6, 7, 8, 9, 10, 11, 12, 13, 14, 15, 16, 17, 18, 19, or 20 miRNA(s)) is selected from the group consisting of SEQ ID NOs: 25, 26, 38, 41, 43, 44, 45, 47, 98, 100, 107, 126, 145, 147, 148, 150, 165, 168, 171, 212, and a sequence having at least 90%, preferably at least 95%, more preferably at least 99%, i.e. at least 90, 91, 92, 93, 94, 95, 96, 97, 98, or 99%, sequence identity thereto.

In addition to the at least one miRNA selected from the group consisting of SEQ ID NO: 1 to SEQ ID NO: 702, and a sequence having at least 90%, preferably at least 95%, more preferably at least 99%, i.e. at least 90, 91, 92, 93, 94, 95, 96, 97, 98, or 99%, sequence identity thereto, at least one further miRNA selected from the group consisting of SEQ ID NO: 703 to SEQ ID NO: 710, and a sequence having at least 90%, preferably at least 95%, more preferably at least 99%, i.e. at least 90, 91, 92, 93, 94, 95, 96, 97, 98, or 99%, sequence identity thereto is preferably used.

As to the biological sample and/or the individual, it is referred to third aspect and/or to the definition part of the present invention.

The invention is summarized as follows:
1. A method for diagnosing Parkinson's disease (PD) in an individual (suspected of having PD) comprising the step of:
   determining the level of at least one miRNA selected from the group consisting of SEQ ID NO: 1 to SEQ ID NO: 702 and a sequence having at least 90% sequence identity thereto in a biological sample isolated from the individual (suspected of having PD).
2. The method of item 1, wherein the level of the at least one miRNA is compared to a reference level of said at least one miRNA.
3. The method of item 2, wherein the reference level is the level determined by measuring at least one reference biological sample isolated from at least one subject not suffering from PD.
4. The method of item 3, wherein
   (a) the level of the at least one miRNA selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299,301,305,310,311,312,317,319,320,321,323,324,326,332,336,339,341, 342, 343, 344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90% sequence identity thereto below the reference level indicates that the individual has PD, and/or
   (b) the level of the at least one miRNA selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90% sequence identity thereto above the reference level indicates that the individual has PD.
5. A method for determining the course of Parkinson's disease (PD) in an individual having PD comprising the step of:
   determining the level of at least one miRNA selected from the group consisting of SEQ ID NO: 1 to SEQ ID NO: 702 and a sequence having at least 90% sequence identity thereto in a biological sample isolated from the individual (having PD).
6. The method of item 5, wherein the level of the at least one miRNA is compared to a reference level of said at least one miRNA.
7. The method of item 6, wherein the reference level is the level determined by measuring at least one reference biological sample isolated from
   at least one subject not suffering from PD, or
   at least one subject suffering from PD.
8. The method of any one of items 5 to 7, wherein said determining comprises determining the level of the at least one miRNA in a biological sample at a first point in time and in at least one further biological sample at a later point in time and comparing said levels determined at the different time points.
9. The method of item 8, wherein
   (a) the at least one miRNA is selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299, 301, 305, 310,311,312,317,319,320,321,323,324,326,332,336,339,341,342,343,344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90% sequence identity thereto and wherein the level of said at least one miRNA which
      (i) decreases over time indicates that PD worsens in the individual,
      (ii) does not change over time indicates that PD does not worsen/is stable in the individual, or
      (iii) increases over time indicates that PD improves in the individual, and/or
   (b) the at least one miRNA is selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90% sequence identity thereto and wherein the level of said at least one miRNA which
      (i) increases over time indicates that PD worsens in the individual,
      (ii) does not change over time indicates that PD does not worsen/is stable in the individual, or
      (iii) decreases over time indicates that PD improves in the individual.
10. The method of any one of items 5 to 9, wherein the individual receives or has received a treatment of PD.
11. The method of item 10, wherein the treatment of PD is selected from the group consisting of the administration of a drug, speech therapy, exercise training, mental training, and physical rehabilitation.
12. A method for determining the risk for developing Parkinson's disease (PD) in an individual comprising the step of:
   determining the level of at least one miRNA selected from the group consisting of SEQ ID NO: 1 to SEQ ID NO: 702 and a sequence having at least 90% sequence identity thereto in a biological sample isolated from the individual (suspected of having PD).
13. The method of item 12, wherein the level of the at least one miRNA is compared to a reference level of said at least one miRNA.
14. The method of item 13, wherein the reference level is the level determined by measuring at least one reference biological sample isolated from at least one subject not suffering from PD.
15. The method of item 14, wherein
   (a) the level of the at least one miRNA selected from the group consisting of SEQ ID NOs: 1, 2, 3, 5, 8, 10, 16, 17, 19, 20, 21, 22, 25, 28, 29, 30, 31, 32, 34, 35, 37, 38, 39, 40, 42, 43, 44, 45, 46, 47, 49, 51, 52, 54, 55, 56, 58, 59, 60, 61, 62, 63, 64, 65, 66, 67, 68, 69, 70, 71, 72, 73, 74, 75, 76, 77, 78, 79, 80, 81, 83, 85, 87, 89, 92, 93, 94, 97, 102, 104, 105, 106, 108, 109, 113, 122, 123, 127, 128, 134, 138, 143, 146, 149, 158, 163, 164, 166, 170, 177, 178, 183, 184, 185, 187, 189, 190, 195, 204, 206, 211, 215, 216, 221, 223, 225, 226, 227, 228, 229, 235, 237, 240, 241, 244, 245, 247, 249, 253, 254, 256, 259, 267, 268, 269, 272, 276, 277, 280, 284, 285, 291, 292, 294, 299,301,305,310,311,312,317,319,320,321,323,324,326,332,336,339,341, 342, 343, 344, 345, 347, 354, 356, 359, 360, 361, 364, 365, 366, 369, 372, 377, 378, 379, 380, 381, 383, 384, 385, 387, 390, 391, 392, 393, 395, 396, 397, 400, 401, 403, 404, 407, 408, 410, 411, 414, 415, 417, 420, 423, 430, 437, 438, 439, 440, 442, 447, 448, 449, 452, 453, 457, 459, 460, 461, 465, 466, 468, 469, 474, 476, 477, 485, 492, 493, 494, 495, 499, 501, 502, 504, 505, 510, 511, 519, 520, 521, 525, 526, 527, 530, 531, 532, 533, 534, 538, 539, 544, 545, 547, 548, 549, 555, 556, 557, 558, 567, 568, 570, 574, 575, 576, 586, 587, 594, 595, 597, 599, 604, 605, 608, 611, 612, 615, 616, 617, 619, 620, 624, 625, 626, 634, 635, 637, 638, 639, 642, 643, 644, 645, 646, 647, 648, 649, 651, 653, 654, 655, 658, 660, 661, 662, 663, 664, 665, 670, 673, 678, 680, 684, 691, 692, 697, 699, and a sequence having at least 90% sequence identity thereto below the reference level indicates that the individual is at risk for developing PD, and/or
   (b) the level of the at least one miRNA selected from the group consisting of SEQ ID NOs: 4, 6, 7, 9, 11, 12, 13, 14, 15, 18, 23, 24, 26, 27, 33, 36, 41, 48, 50, 53, 57, 82, 84, 86, 88, 90, 91, 95, 96, 98, 99, 100, 101, 103, 107, 110, 111, 112, 114, 115, 116, 117, 118, 119, 120, 121, 124, 125, 126, 129, 130, 131, 132, 133, 135, 136, 137, 139, 140, 141, 142, 144, 145, 147, 148, 150, 151, 152, 153, 154, 155, 156, 157, 159, 160, 161, 162, 165, 167, 168, 169, 171, 172, 173, 174, 175, 176, 179, 180, 181, 182, 186, 188, 191, 192, 193, 194, 196, 197, 198, 199, 200, 201, 202, 203, 205, 207, 208, 209, 210, 212, 213, 214, 217, 218, 219, 220, 222, 224, 230, 231, 232, 233, 234, 236, 238, 239, 242, 243, 246, 248, 250, 251, 252, 255, 257, 258, 260, 261, 262, 263, 264, 265, 266, 270, 271, 273, 274, 275, 278, 279, 281, 282, 283, 286, 287, 288, 289, 290, 293, 295, 296, 297, 298, 300, 302, 303, 304, 306, 307, 308, 309, 313, 314, 315, 316, 318, 322, 325, 327, 328, 329, 330, 331, 333, 334, 335, 337, 338, 340, 346, 348, 349, 350, 351, 352, 353, 355, 357, 358, 362, 363, 367, 368, 370, 371, 373, 374, 375, 376, 382, 386, 388, 389, 394, 398, 399, 402, 405, 406, 409, 412, 413, 416, 418, 419, 421, 422, 424, 425, 426, 427, 428, 429, 431, 432, 433, 434, 435, 436, 441, 443, 444, 445, 446, 450, 451, 454, 455, 456, 458, 462, 463, 464, 467, 470, 471, 472, 473, 475, 478, 479, 480, 481, 482, 483, 484, 486, 487, 488, 489, 490, 491, 496, 497, 498, 500, 503, 506, 507, 508, 509, 512, 513, 514, 515, 516, 517, 518, 522, 523, 524, 528, 529, 535, 536, 537, 540, 541, 542, 543, 546, 550, 551, 552, 553, 554, 559, 560, 561, 562, 563, 564, 565, 566, 569, 571, 572, 573, 577, 578, 579, 580, 581, 582, 583, 584, 585, 588, 589, 590, 591, 592, 593, 596, 598, 600, 601, 602, 603, 606, 607, 609, 610, 613, 614, 618, 621, 622, 623, 627, 628, 629, 630, 631, 632, 633, 636, 640, 641, 650, 652, 656, 657, 659, 666, 667, 668, 669, 671, 672, 674, 675, 676, 677, 679, 681, 682, 683, 685, 686, 687, 688, 689, 690, 693, 694, 695, 696, 698, 700, 701, 702, and a sequence having at least 90% sequence identity thereto above the reference level indicates that the individual is at risk for developing PD.
16. The method of any one of items 1 to 15, wherein the biological sample is a blood sample.
17. The method of item 16, wherein the blood sample is selected from the group consisting of whole blood and a blood cellular fraction.
18. The method of item 17, wherein the blood cellular fraction comprises erythrocytes, leukocytes, and thrombocytes.
19. The method of any one of items 1 to 18, wherein the level is the expression level.
20. Use of at least one polynucleotide for detecting at least one miRNA in a biological sample isolated from an individual for diagnosing Parkinson's disease (PD) in the individual, wherein the at least one miRNA is selected from the group consisting of SEQ ID NO: 1 to SEQ ID NO: 702.
21. Use of at least one polynucleotide for detecting at least one miRNA in a biological sample isolated from an individual having Parkinson's disease (PD) for determining the course of PD in the individual,
   wherein the at least one miRNA is selected from the group consisting of SEQ ID NO: 1 to SEQ ID NO: 702.
22. Use of at least one polynucleotide for detecting at least one miRNA in a biological sample isolated from an individual for determining the risk of the individual to develop Parkinson's disease (PD),
   wherein the at least one miRNA is selected from the group consisting of SEQ ID NO: 1 to SEQ ID NO: 702.
23. The use of any one of items 20 to 22, wherein
   (i) the at least one polynucleotide is at least partially (reverse) complementary, preferably (reverse) complementary, to the at least one miRNA of any one of items 20 to 22, or
   (ii) the at least one polynucleotide has at least 90% sequence identity to the polynucleotide according to (i).
24. The use of any one of items 20 to 23, wherein the biological sample is a blood sample.
25. The use of item 24, wherein the blood sample is selected from the group consisting of whole blood and a blood cellular fraction.
26. The use of item 25, wherein the blood cellular fraction comprises erythrocytes, leukocytes, and thrombocytes.
27. A kit for diagnosing Parkinson's disease (PD) in an individual, for determining the course of Parkinson's disease (PD) in an individual having PD, and/or for determining the risk for developing Parkinson's disease (PD) in an individual comprising:
   (i) means for determining the level of at least one miRNA selected from the group consisting of SEQ ID NO: 1 to SEQ ID NO: 702 and a sequence having at least 90% sequence identity thereto in a biological sample isolated from the individual, and
   (ii) optionally at least one reference.
28. The kit of item 27, wherein the means for determining the level of the at least one miRNA in a biological sample isolated from the individual comprise at least one polynucleotide as defined in any one of items 20 to 26.
29. The kit of items 27 or 28, wherein the kit is useful for conducting the methods according to any one of items 1 to 19.
30. The kit of any one of items 27 to 29, wherein the kit further comprises
   (iii) a container, and/or
   (iv) a data carrier.
31. The kit of item 30, wherein the data carrier comprises instructions on how to carry out the methods according to any one of items 1 to 19.

Various modifications and variations of the invention will be apparent to those skilled in the art without departing from the scope of invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art in the relevant fields are intended to be covered by the present invention.

### BRIEF DESCRIPTION OF THE FIGURES

The following Figures are merely illustrative of the present invention and should not be construed to limit the scope of the invention as indicated by the appended claims in any way.

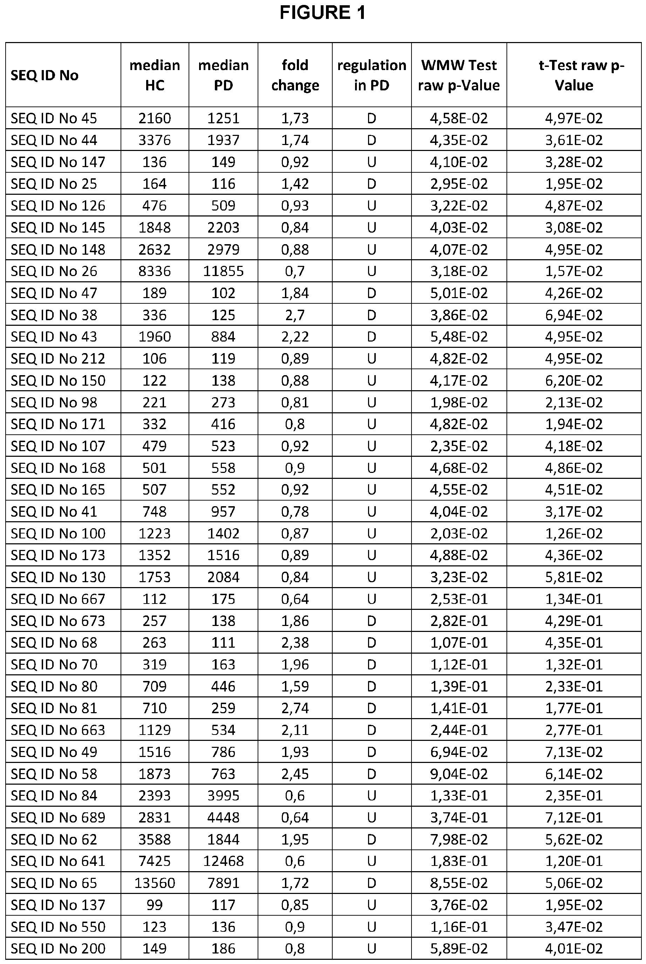

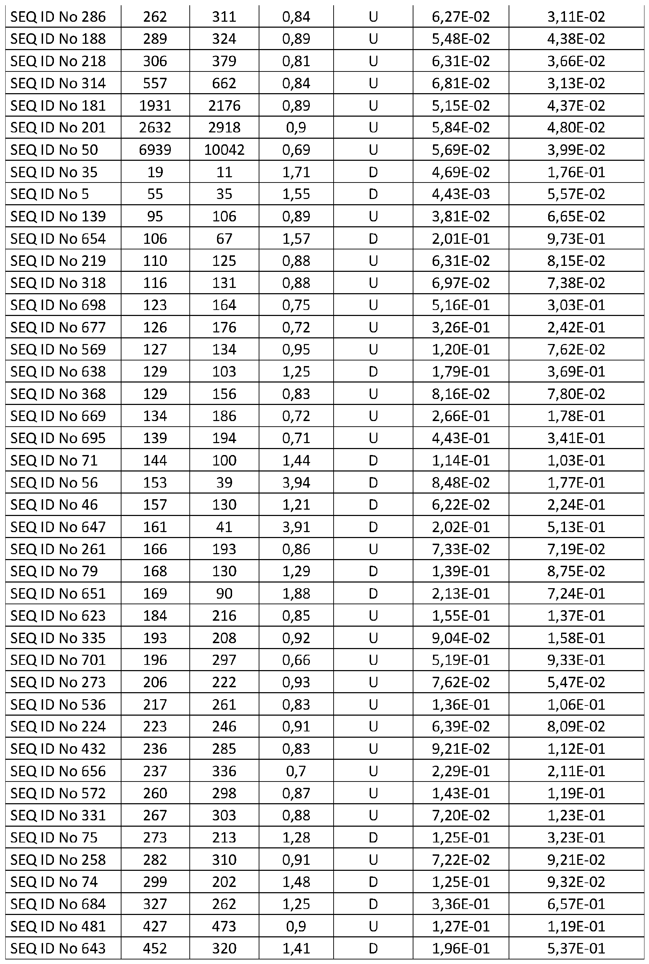

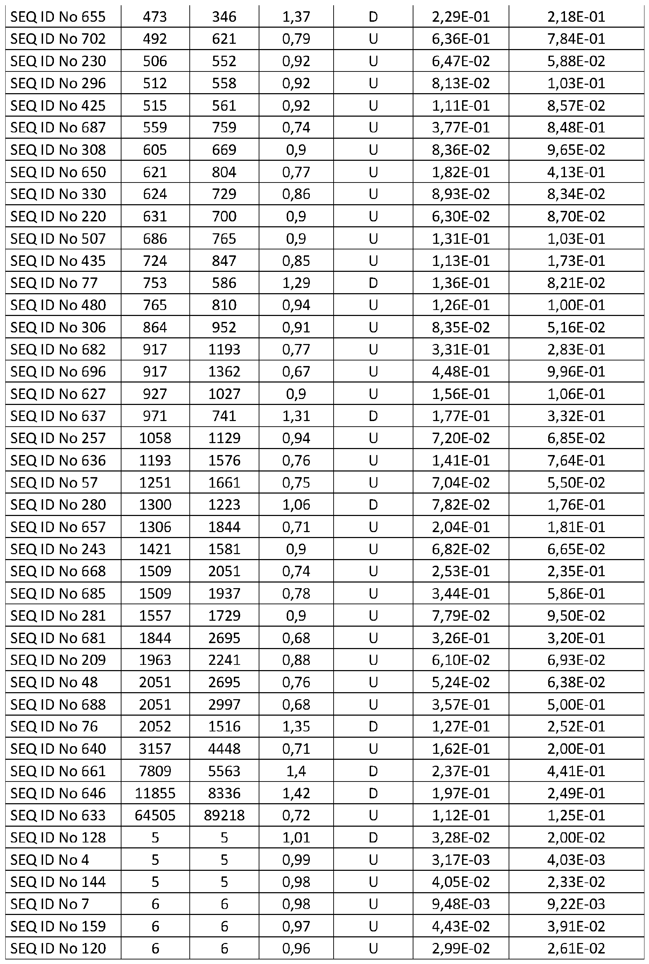

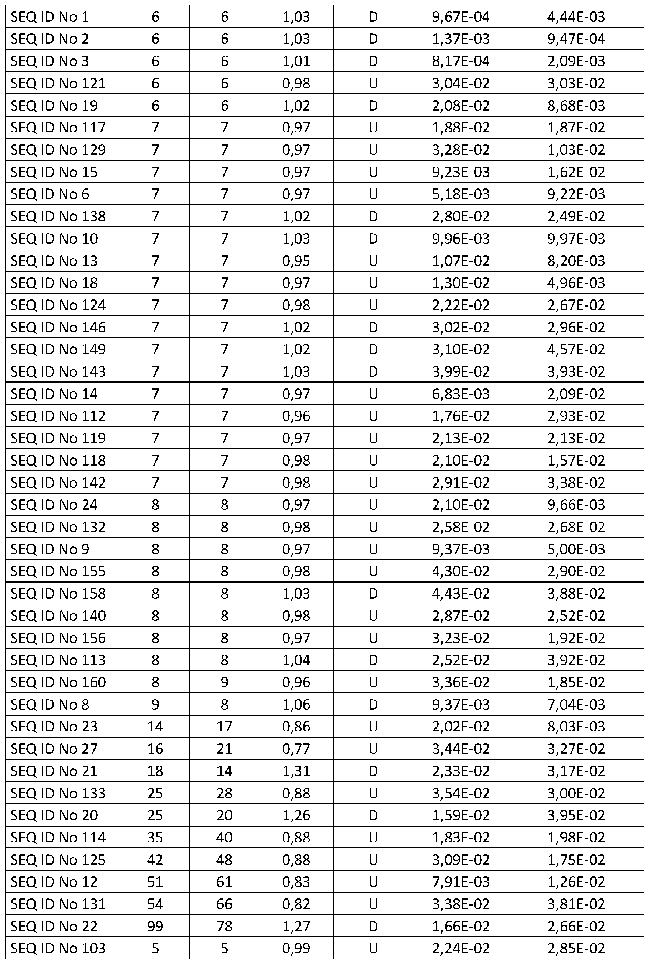

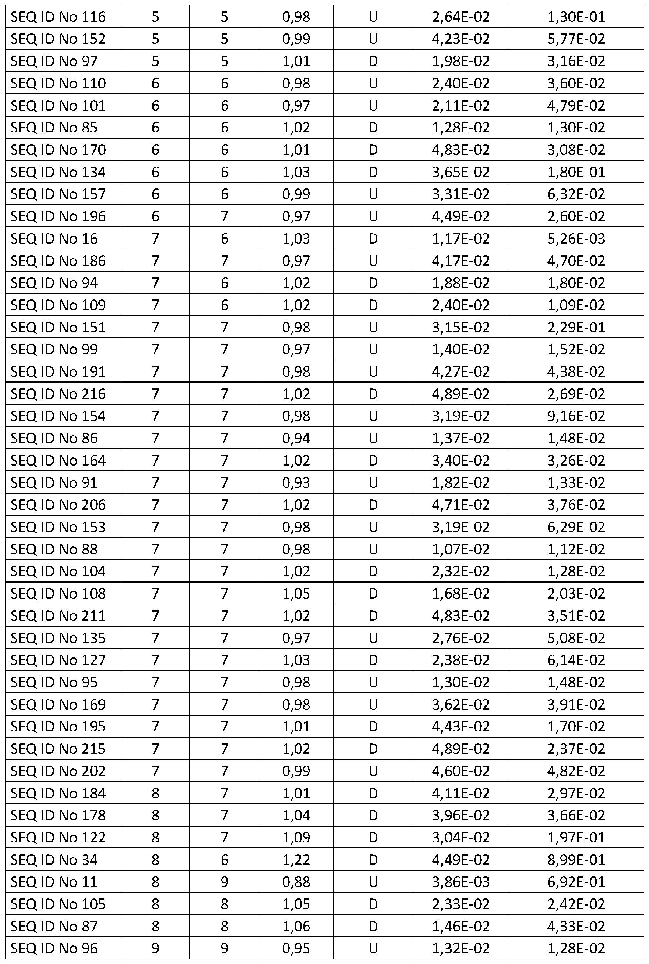

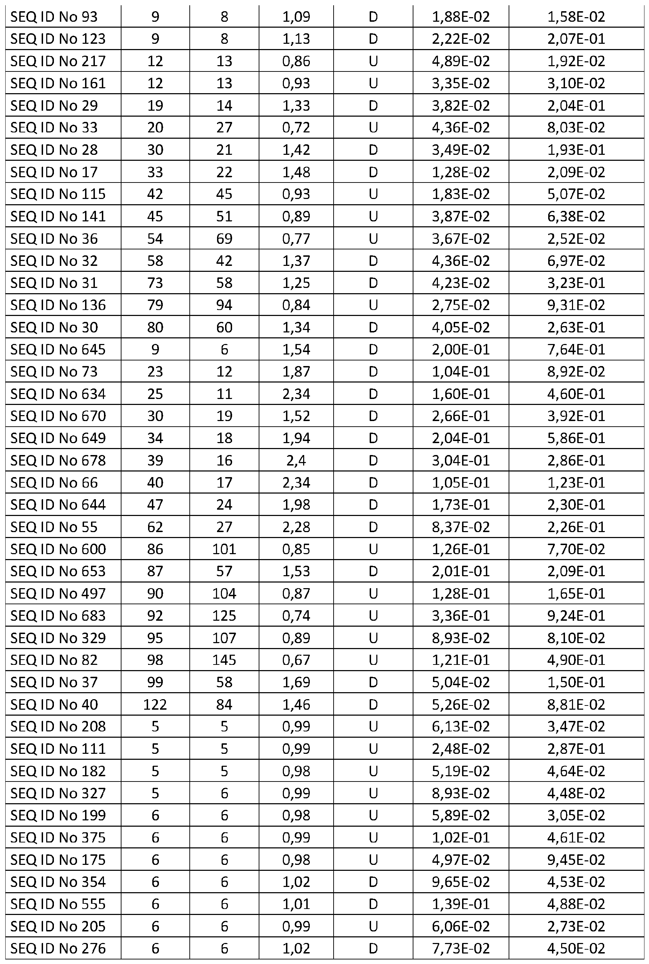

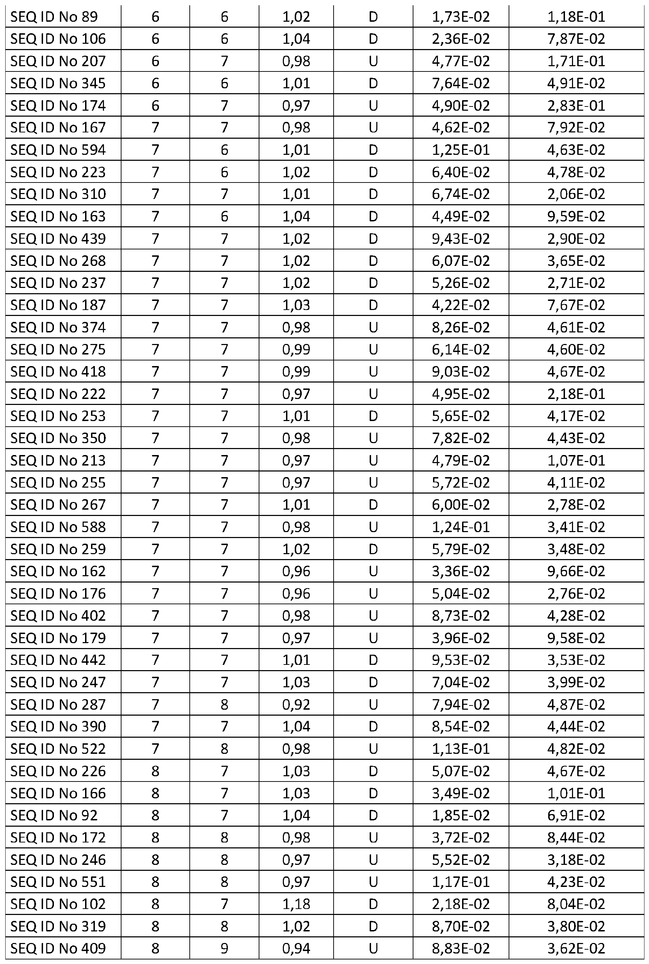

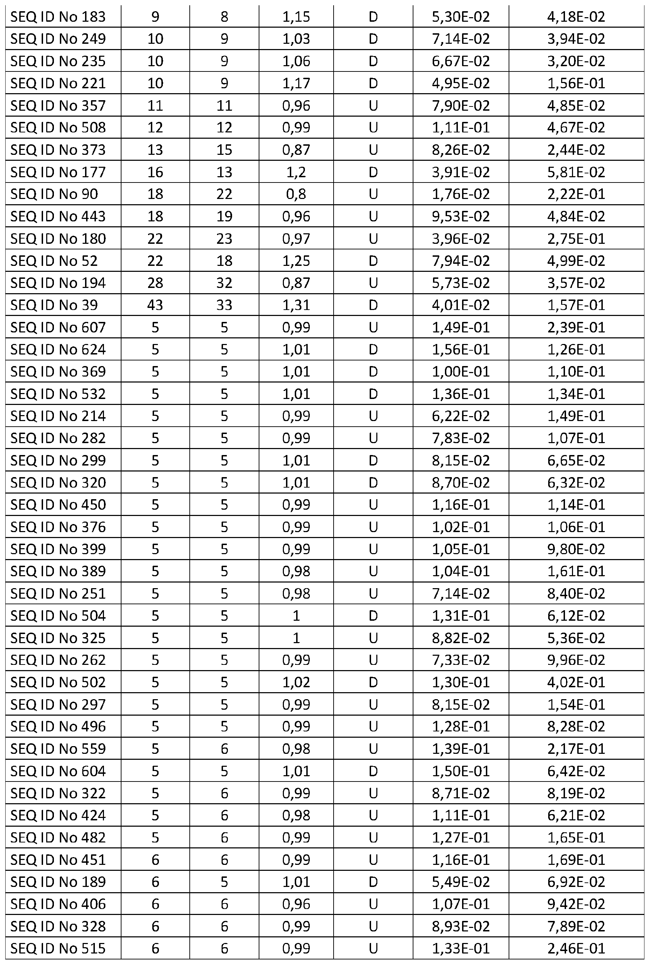

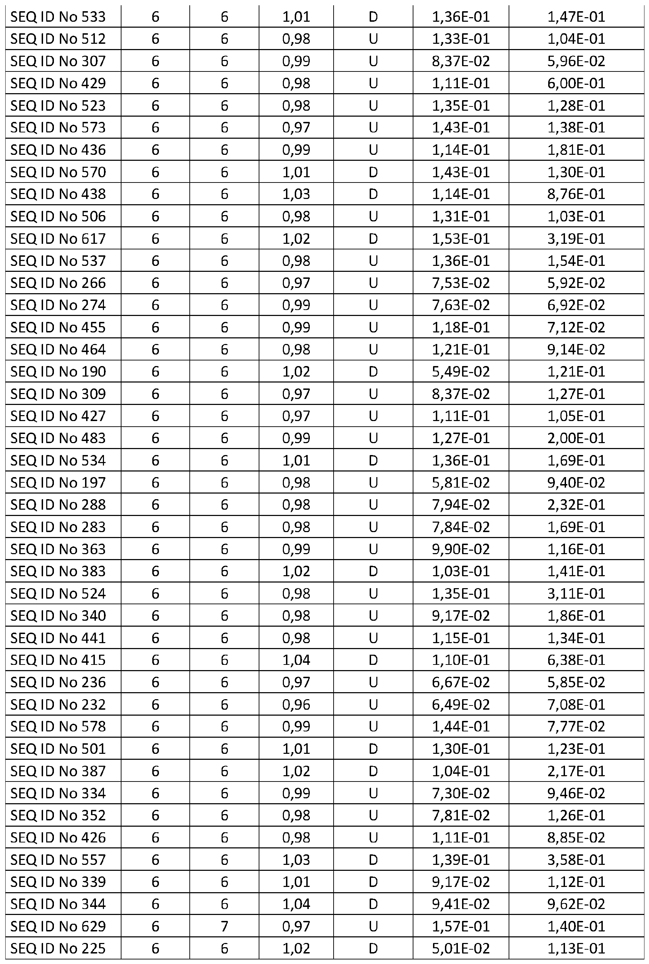

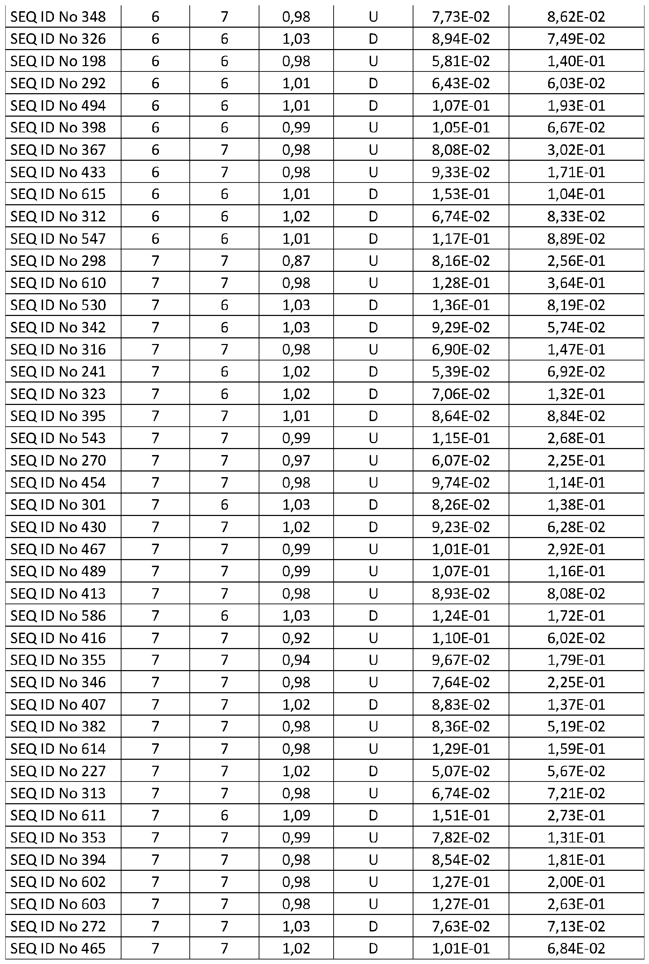

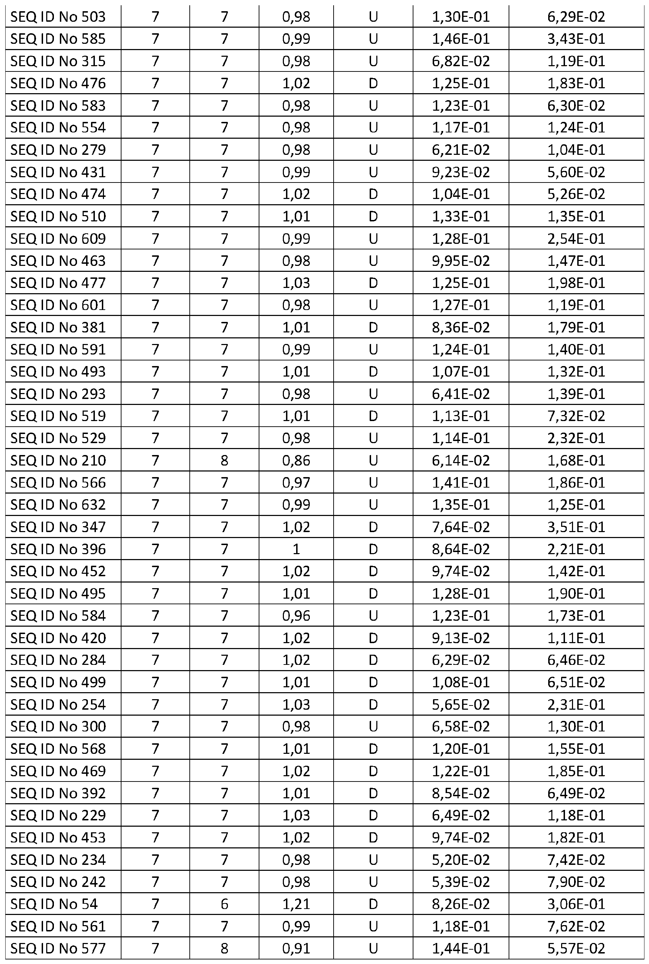

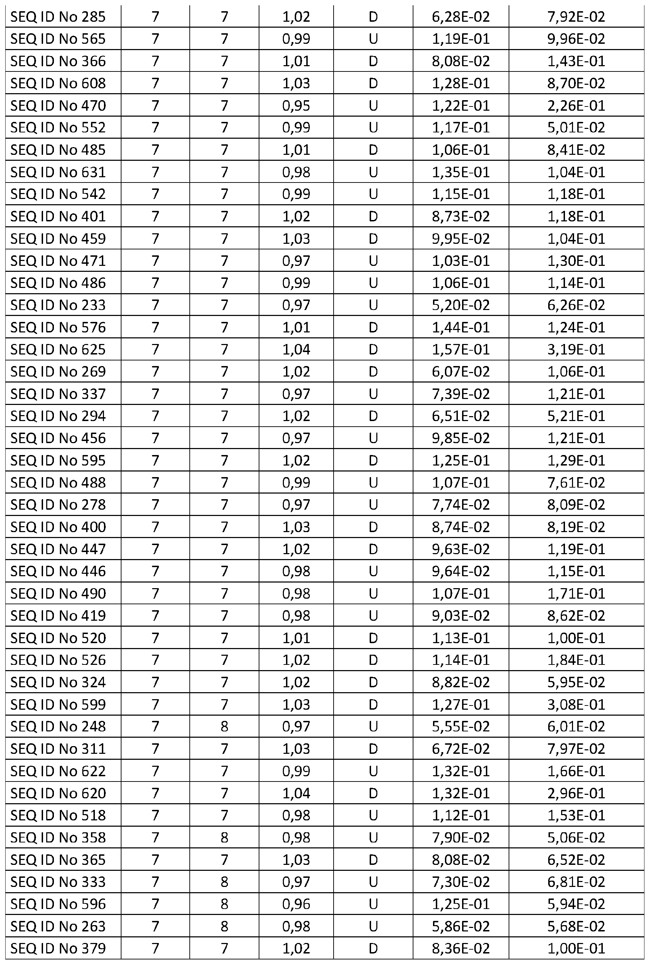

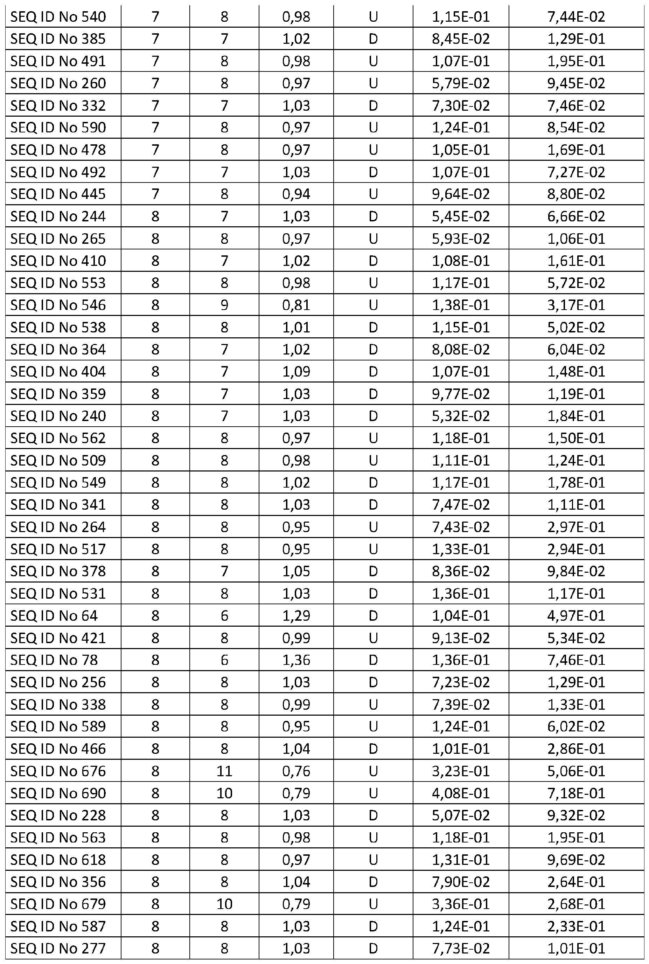

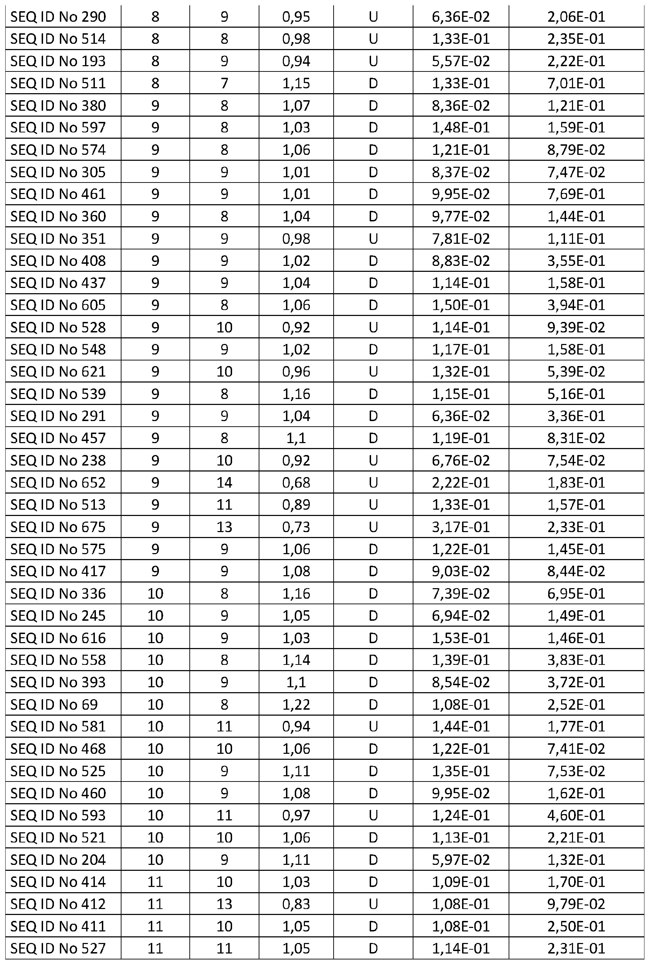

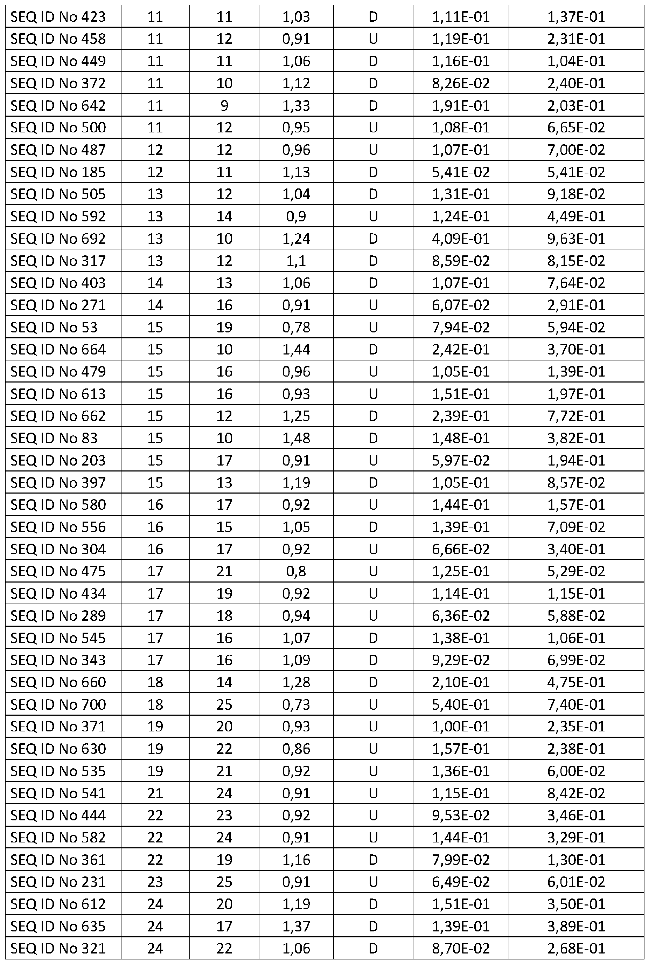

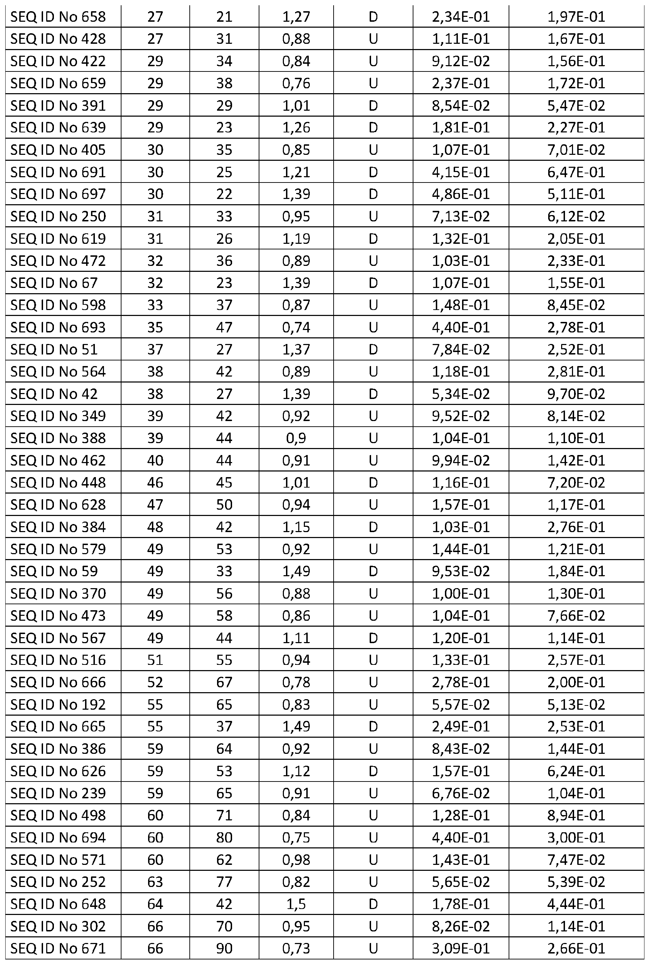

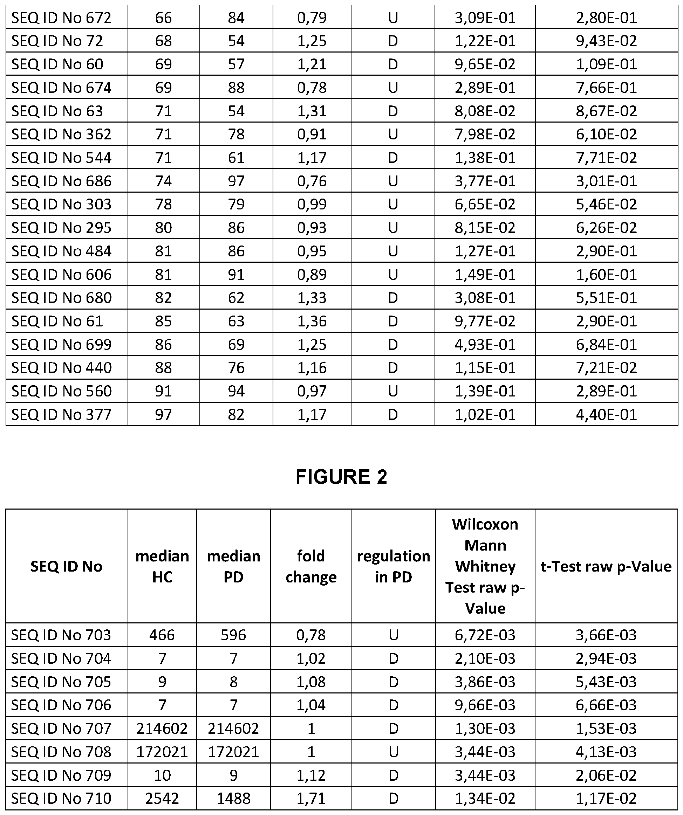

**Figure 1****:** Overview of miRNAs that are newly found to be differentially regulated between healthy controls and individuals suffering from Parkinson's disease (PD). Experimental details: SEQ ID NO: sequence identification number of the respective miRNA, median HC: median intensity obtained from microarray analysis for healthy controls (HC), median PD: median intensity obtained from microarray analysis for individuals suffering from Parkinson's disease (PD), fold-change: ratio of median HC/median PD, D: downregulated in Parkinson's disease (PD) compared to healthy controls, U: upregulated in Parkinson's disease (PD) compared to healthy controls, WMW test raw p-value: p-value obtained when applying WMW test, and t-test raw p-value: p-value obtained when applying t-test.
**Figure 2****:** Overview of miRNAs that are known to be differentially regulated between healthy controls and individuals suffering from Parkinson's disease (PD). Experimental details: SEQ ID NO: sequence identification number of the respective miRNA, median HC: median intensity obtained from microarray analysis for healthy controls (HC), median PD: median intensity obtained from microarray analysis for individuals suffering from Parkinson's disease (PD), fold-change: ratio of median HC/median PD, D: downregulated in Parkinson's disease (PD) compared to healthy controls, U: upregulated in Parkinson's disease (PD) compared to healthy controls, WMW test raw p-value: p-value obtained when applying WMW test, and t-test raw p-value: p-value obtained when applying t-test.

### EXAMPLES

The examples given below are for illustrative purposes only and do not limit the invention described above in any way.

### Example

### Identification of miRNAs as biomarkers for the diagnosis of Parkinson's disease (PD) in an individual or for determining the course of Parkinson's disease (PD) in an individual suffering from PD

### 1. Materials and methods

### 1.1 Patient samples

In the present study, patients undergoing significant surgical interventions were included. Blood samples (2.5 mL per patient) were collected in PAXgene tubes prior to and following surgery in time intervals (3 days). In the present study patients with the clinical diagnosis of PD have been included. These preferentially contain de-novo Parkinson patients that have been newly diagnosed with PD. Respective patients are usually not undergoing Parkinson therapies yet. Controls are age and gender matched individuals without Parkinson symptoms.

### 1.2 Sample preparation

Prior to RNA extraction, PAXgene tubes were thawed overnight at room temperature to ensure complete lysis of blood cells. Total RNA, including miRNA, was extracted and purified using the PAXgene Blood miRNA Kit in accordance with the manufacturer's instructions (Qiagen GmbH, Hilden, Germany). Quantification of purified RNA was performed with NanoDrop 1000 (Thermo Fisher Scientific, Waltham, Massachusetts, USA). The quality and integrity of the RNA (RIN value) was evaluated using Agilent Bioanalyzer and the Nano RNA Kit in accordance with the manufacturer's protocols (Agilent Technologies, Santa Clara, California, USA).

### 1.3 Sample measurement

For miRNA expression, profiling samples were analysed on Agilent Sureprint G3 Human miRNA (8×60k) microarray slides with the latest miRBase v21 content. Each array targets 2,549 microRNAs with 20 replicates per probe. Extracted miRNA was labeled and hybridized using the miRNA Complete Labeling and Hybridization Kit from Agilent, in accordance with the manufacturer's protocol (Agilent Technologies, Santa Clara, California, USA). After rotating hybridization for 20 hours at 55 C, the slides were washed twice and scanned on Agilent's SureScan Microarray Scanner. Image files from the scanner were transformed into text raw data using Feature Extraction Software (Agilent Technologies) for bioinformatics analysis.

Further, the same samples were analysed on a second microarray with proprietary miRNA content. This microarray is entitled as "all human miRNA blood microarray", manufactured by Agilent (Agilent Technologies, Santa Clara, California, USA) and distributed by Hummingbird Diagnostics GmbH (Heidelberg, Germany). This array contains in addition to the miRBase miRNAs that are expressed in blood also 1,727 miRNAs that are not contained in the miRBase. This microarray is processed using the same methods as the original Agilent microarrays (see above) and is thought to be a general diagnostic array to find pathologies from blood samples and other body fluids.

### 1.4 Data analysis, statistics

For data processing, the profiled samples were subjected to normalization. The 2,549 human miRNAs available on the Agilent miRBase v21 arrays were then used for the bioinformatics analysis. Similarly, the 1,7272 new miRNAs were normalized and evaluated. For subsequent data analysis different methods were applied (e.g. unsupervised clustering or analysis of variance). For pairwise comparisons, the t-test was used for comparisons between the control group and the other classes. In addition, multiple comparison was also carried out using the analysis of variance (ANOVA) test. Because of the nature of the study and to make p-values between both microarrays (known content from miRBase and new content from the "all human miRNA blood microarray"), the p-values are reported as unadjusted p-values.

### 2. Results

The markers with SEQ ID NO: 1 to SEQ ID NO: 710 have been found to be differentially regulated between PD and HC subjects. The criteria for considering a miRNA to have sufficient diagnostic power include in this case: "t-test p-value below an alpha level of 0.05" OR "WMW-test p-value below an alpha level of 0.05" OR "at least 20% change in the expression level between the two groups" OR "AUC above 0.6".

Selected examples of miRNA biomarkers for Parkinson's disease (PD) identified in this study are shown in **Figures 1** and **2**.

## Claims

1. A method for diagnosing Parkinson's disease (PD) in an individual suspected of having PD comprising the step of:
determining the level of at least one miRNA selected from the group consisting of SEQ ID NO: 62, SEQ ID NO: 651, SEQ ID NO: 356, and a sequence having at least 90% sequence identity thereto in a blood sample isolated from the individual suspected of having PD.

2. The method of claim 1,
wherein the level of the at least one miRNA is compared to a reference level of said at least one miRNA,
wherein preferably the reference level is the level determined by measuring at least one reference biological sample isolated from at least one subject not suffering from PD, and
wherein more preferably the level of the at least one miRNA below the reference level indicates that the individual has PD.

3. A method for determining the course of Parkinson's disease (PD) in an individual having PD comprising the step of:
determining the level of at least one miRNA selected from the group consisting of SEQ ID NO: 62, SEQ ID NO: 651, SEQ ID NO: 356, and a sequence having at least 90% sequence identity thereto in a blood sample isolated from the individual having PD.

4. The method of claim 3,
wherein the level of the at least one miRNA is compared to a reference level of said at least one miRNA,
wherein preferably the reference level is the level determined by measuring at least one reference biological sample isolated from
at least one subject not suffering from PD, or
at least one subject suffering from PD.

5. The method of any one of claims 3 or 4, wherein said determining comprises determining the level of the at least one miRNA in a biological sample at a first point in time and in at least one further biological sample at a later point in time and comparing said levels determined at the different time points.

6. The method of claim 5, wherein the level of said at least one miRNA which
(i) decreases over time indicates that PD worsens in the individual,
(ii) does not change over time indicates that PD does not worsen/is stable in the individual, or
(iii) increases over time indicates that PD improves in the individual.

7. The method of any one of claims 3 to 6,
wherein the individual receives or has received a treatment of PD,
wherein preferably the treatment of PD is selected from the group consisting of the administration of a drug, speech therapy, exercise training, mental training, and physical rehabilitation.

8. A method for determining the risk for developing Parkinson's disease (PD) in an individual comprising the step of:
determining the level of at least one miRNA selected from the group consisting of SEQ ID NO: 62, SEQ ID NO: 651, SEQ ID NO: 356, and a sequence having at least 90% sequence identity thereto in a blood sample isolated from the individual suspected of having PD.

9. The method of claim 8,
wherein the level of the at least one miRNA is compared to a reference level of said at least one miRNA,
wherein preferably the reference level is the level determined by measuring at least one reference biological sample isolated from at least one subject not suffering from PD, wherein more preferably the level of the at least one miRNA below the reference level indicates that the individual is at risk for developing PD.

10. Use of at least one polynucleotide for detecting at least one miRNA in a blood sample isolated from
an individual for diagnosing Parkinson's disease (PD) in the individual,
an individual having Parkinson's disease (PD) for determining the course of PD in the individual, or
an individual for determining the risk of the individual to develop Parkinson's disease (PD),
wherein the at least one miRNA is selected from the group consisting of SEQ ID NO: 62, SEQ ID NO: 651, and SEQ ID NO: 356.

11. The use of claim 10, wherein
(i) the at least one polynucleotide is at least partially (reverse) complementary, preferably (reverse) complementary, to the at least one miRNA of claim 10, or
(ii) the at least one polynucleotide has at least 90% sequence identity to the polynucleotide according to (i).

12. A kit for diagnosing Parkinson's disease (PD) in an individual, for determining the course of Parkinson's disease (PD) in an individual having PD, and/or for determining the risk for developing Parkinson's disease (PD) in an individual comprising:
(i) means for determining the level of at least one miRNA selected from the group consisting of SEQ ID NO: 62, SEQ ID NO: 651, SEQ ID NO: 356, and a sequence having at least 90% sequence identity thereto in a blood sample isolated from the individual, and
(ii) optionally at least one reference.

13. The kit of claim 12, wherein the means for determining the level of the at least one miRNA in a biological sample isolated from the individual comprise at least one polynucleotide as defined in claims 10 or 11.

14. The method of claims 1 to 9, the use of claims 10 or 11, or the kit of claims 12 or 13, wherein the blood sample is selected from the group consisting of whole blood and a blood cellular fraction.

15. The method of claims 1 to 9, the use of claims 10 or 11, or the kit of claims 12 or 13, wherein the blood sample is a blood preparation produced from whole blood by removing the extracellular fraction.
